# EUROPEAN PATENT APPLICATION

(11) **EP 3 569 603 A1**
(43) Date of publication of application: **20.11.2019**
(21) Application number: 18172191.1
(22) Date of filing: 14.05.2018
(51) Int. Cl.: C07D 493/04, H01L 51/00

(54) **POLYCYCLIC COMPOUND, AND ORGANIC ELECTROLUMINESCENCE ELEMENT COMPRISING THE POLYCYCLIC COMPOUND**

(71) Applicant: Idemitsu Kosan Co., Ltd., Chiyoda-ku, Tokyo 100-8321 (JP)
(72) Inventor: Groarke, Michelle, 4125 Riehen (CH); Raimann, Thomas, 4334 Sisseln (CH); Chebotareva, Natalia, 68220 Hagenthal le Bas (FR); Kawamura, Masahiro, 4057 Basel (CH)
(74) Representative: Hollah, Dorothee

(57) **Abstract**

A polycyclic compound represented by one of the following formulae 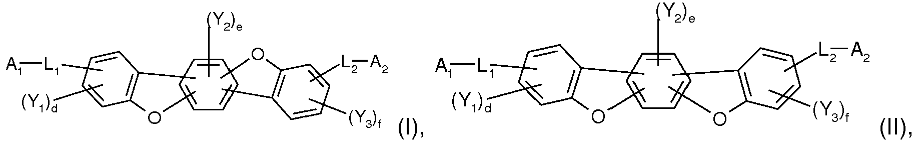 where:
A₁ represents a group of formula (V) 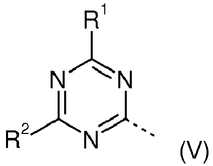 and
A₂ represents a group of formula (VI) or (VII) 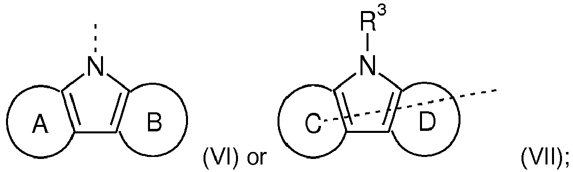 an organic electroluminescence device comprising a cathode, an anode, and a plurality of organic layers provided between the cathode and the anode, wherein at least one of the plurality of organic layers is an emitting layer, wherein at least one organic layer comprises at least one inventive polycyclic compound; and the use of the inventive polycyclic compound in an organic electroluminescence device.

## Description

The present invention relates to a polycyclic compound, and an organic electroluminescence device using the same.

An organic electroluminescence device (hereinafter, "electroluminescence" may be abbreviated as "EL") is a spontaneous light emitting device which utilizes the principle that a luminescent substance emits light by energy of recombination of holes injected from an anode and electrons injected from a cathode when an electric field is applied. Since an organic EL device of the laminate type driven under a low electric voltage was reported, many studies have been conducted on organic EL devices using organic materials as the constituent materials. Typical devices of the laminate type use tris(8-quinolinolato) aluminum for a light emitting layer and a triphenylene-diamine derivative for a hole transporting layer. Advantages of the laminate structure are that the efficiency of hole injection into the light emitting layer can be increased, that the efficiency of forming excitons which are formed by blocking and recombining electrons injected from the cathode can be increased, and that excitons formed within the light emitting layer can be enclosed. As described above, for the structure of the organic EL device, a two-layered structure having a hole transporting (injecting) layer and an electron transporting light emitting layer and a three-layered structure having a hole transporting (injecting) layer, a light emitting layer, and an electron transporting (injecting) layer are well known. To increase the efficiency of recombination of injected holes and electrons in the devices of the laminate type, the structure of the device and the process for forming the device have been studied.

As the light emitting material of the organic EL device, chelate complexes such as tris (8-quinolinolato) aluminum complexes, coumarine derivatives, tetraphenylbutadiene derivatives, distyrylarylene derivatives, and oxadiazole derivatives are known. It is reported that light in the visible region ranging from blue light to red light can be obtained by using these light emitting materials, and development of a device exhibiting color images is expected.

In addition, it has been more recently proposed that a phosphorescent material as well as a fluorescent material be utilized in the light emitting layer of an organic EL device. High luminous efficiency is achieved by utilizing the singlet and triplet states of an excited state of an organic phosphorescent material in the light emitting layer of an organic EL device. Upon recombination of an electron and a hole in an organic EL device, singlet excitons and triplet excitons may be produced at a ratio of 1:3 owing to a difference in spin multiplicity between the singlet and triplet excitons, so the use of phosphorescent material may achieve luminous efficiency three to four times as high as that of a device using fluorescence alone.

The following patent literatures each describe such materials for an organic EL device.

WO 2006/122630 A1 describes a compound using a structure obtained by crosslinking a terphenylene skeleton with, for example, a carbon atom, nitrogen atom, or oxygen atom as a mother skeleton. The document, which mainly discloses data indicative of the potential of the compound to serve as a hole transporting material, describes that the compound is used as a host material for a phosphorescent material in a light emitting layer. However, the description is limited to a red phosphorescent device, and the luminous efficiency of the device is not high enough for the device to be put into practical use.

WO 2009/148015 A1 describes a polycyclic compound having an n-conjugated heteroacene skeleton crosslinked with a carbon atom, a nitrogen atom, an oxygen atom, or a sulfur atom, and a halogen compound which can be used for the synthesis of the polycyclic compound. Also provided are an organic EL device which has one or more organic thin film layers including a light emitting layer between a cathode and an anode, at least one layer of the organic thin film layers containing a material for an organic EL device, and a halogen compound serving as an intermediate for the polycyclic compound.

WO 2015/169412 A1 describes carbazole, dibenzofuran, dibenzothiophene and fluorene derivatives which are substituted with electron-deficient heteroaryl groups, in particular for use as triplet matrix materials in organic light emitting devices. WO 2015/169412 further relates to a method for producing said derivatives, and to electronic devices comprising the same.

The present invention has been made with a view to solving the above-mentioned problems, and an object of the present invention is to provide an organic EL device having a good overall performance, especially improved efficiency and/or lifetime, and a polycyclic compound for realizing the same.

The inventors of the present invention have made extensive studies to achieve the object. As a result, the inventors have found that the use of a polycyclic compound represented by the following formula (I) or (II) as a material for an organic EL device achieves the object. Thus, the inventors have completed the present invention.

That is, the present invention provides a polycyclic compound represented by the following formula (I) or (II): 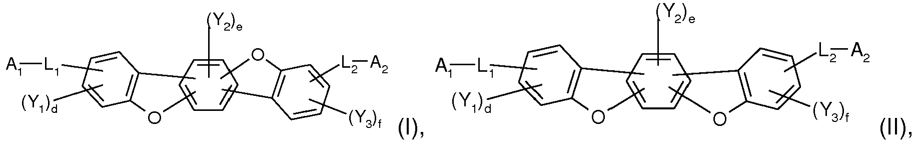 where:
L₁ represents a single bond, a substituted or unsubstituted alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted divalent aromatic hydrocarbon group having a ring formed of 6 to 24 carbon atoms, or a substituted or unsubstituted divalent aromatic heterocyclic group which has a ring formed of 3 to 24;
L₂ represents a single bond, a substituted or unsubstituted alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted divalent aromatic hydrocarbon group having a ring formed of 6 to 24 carbon atoms, or a substituted or unsubstituted divalent aromatic heterocyclic group which has a ring formed of 3 to 24;
A₁ represents a group of formula (V) 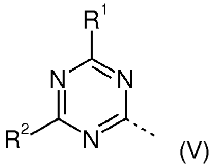 where:
   R¹ and R² each independently represent hydrogen, a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms;
   wherein the dotted line is a bonding site to L₁;
A₂ represents a group of formula (VI) or (VII) 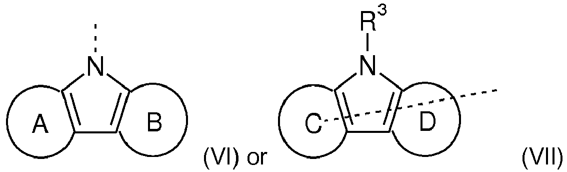 where
   the ring A, the ring B, the ring C and the ring D each independently represent a substituted or unsubstituted monocyclic, bicyclic, or tricyclic carbocyclic ring system formed of 4 to 30 carbon atoms or a substituted or unsubstituted monocyclic, bicyclic, or tricyclic heterocyclic ring system formed of 3 to 30 atoms;
   wherein the dotted line is a bonding site to L₂;
   R³ represents a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms;
   Y₁, Y₂, and Y₃ each independently represent a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms or CN;
   d and f each represent a number of 0, 1, 2, or 3, and e represents a number of 0, 1, or 2.

Further, the present invention provides an organic electroluminescence (EL) device comprising a cathode, an anode, and a plurality of organic layers provided between the cathode and the anode, wherein at least one of the plurality of organic layers is an emitting layer, wherein at least one organic layer comprises at least one compound of formula (I) or (II).

Further, the material for an organic EL device is effective also as a material for an organic electron device such as an organic solar cell, organic semiconductor laser, a sensor using organic matter, or an organic TFT.

According to the present invention, there can be provided an organic EL device having a good overall performance, especially improved efficiency and/or lifetime, and a polycyclic compound for realizing the same.

A material for an organic EL device of the present invention is represented by the following formula (1) or (2). 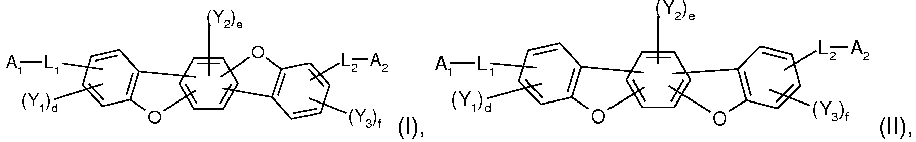 where:
L₁ represents a single bond, a substituted or unsubstituted alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted divalent aromatic hydrocarbon group having a ring formed of 6 to 24 carbon atoms, or a substituted or unsubstituted divalent aromatic heterocyclic group which has a ring formed of 3 to 24 atoms; preferably, L₁ represents a single bond or a substituted or unsubstituted, divalent aromatic hydrocarbon group having a ring formed of 6 to 24 carbon atoms, more preferably, L₁ represents a single bond or a phenylene group, a biphenyl-diyl group, a naphthylene group, a phenanthren-diyl group, a triphenyl-diyl group, or a fluoren-diyl group, most preferably, L₁ represents a single bond or a phenylene group, wherein the phenylene group is a 1,4-phenylene group, 1,3-phenylene group or 1,2-phenylene group; further most preferably, L₁ represents a single bond;
L₂ represents a single bond, a substituted or unsubstituted alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted, divalent aromatic hydrocarbon group having a ring formed of 6 to 24 carbon atoms, or a substituted or unsubstituted, divalent aromatic heterocyclic group which has a ring formed of 3 to 24 atoms; preferably, L₂ represents a single bond or a substituted or unsubstituted, divalent aromatic hydrocarbon group having a ring formed of 6 to 24 carbon atoms, more preferably, L₂ represents a single bond or a phenylene group, a biphenyl-diyl group, a naphthylene group, a phenanthren-diyl group, a triphenyl-diyl group, or a fluoren-diyl group, most preferably, L₂ represents a single bond or a phenylene group, wherein the phenylene group is a 1,4-phenylene group, 1,3-phenylene group or 1,2-phenylene group; further most preferably, L₂ represents a single bond;
A₁ represents a group of formula (V) 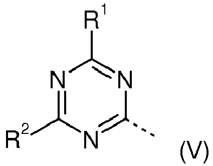 where:
   R¹ and R² each independently represent hydrogen, a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; preferably, R¹ and R² in the group of formula (V) each independently represent a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R¹ and R² each independently represent a substituted or unsubstituted aromatic hydrocarbon group having a ring formed of 6 to 18 carbon atoms, or a substituted or unsubstituted aromatic heterocyclic group which has a ring formed of 3 to 18 atoms, further more preferably R¹ and R² each independently represent phenyl, biphenyl, terphenyl, naphthyl, phenanthrenyl fluorenyl, dibenzofuranyl, dibenzothiophenyl, carbazolyl; even further more preferably R¹ and R² each independently represent phenyl, m-biphenyl, p-biphenyl, 1-naphthyl, 2-naphthyl, 1-fluorenyl, 2-fluorenyl, 3-fluorenyl, 4-fluorenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl, 2-carbazolyl, 3-carbazolyl; most preferably R¹ and R² each independently represent phenyl, m-biphenyl, p-biphenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl; further most preferably R¹ and R² each represent phenyl;
   wherein the dotted line is a bonding site to L₁;
A₂ represents a group of formula (VI) or (VII) 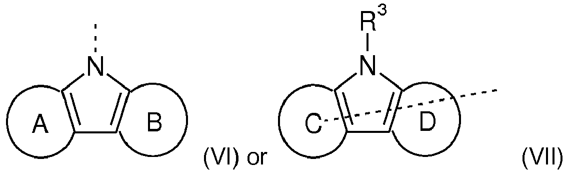 where
   the ring A, the ring B, the ring C and the ring D each independently represent a substituted or unsubstituted monocyclic, bicyclic, or tricyclic carbocyclic ring system formed of 4 to 30 carbon atoms or a substituted or unsubstituted monocyclic, bicyclic, or tricyclic heterocyclic ring system formed of 3 to 30 atoms;
   wherein the dotted line is a bonding site to L₂;
   R³ represents a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; preferably, R³ in the group of formula (V) represents a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R³ represents a substituted or unsubstituted aromatic hydrocarbon group having a ring formed of 6 to 18 carbon atoms, or a substituted or unsubstituted aromatic heterocyclic group which has a ring formed of 3 to 18 atoms, further more preferably R³ represents phenyl, biphenyl, terphenyl, naphthyl, phenanthrenyl, triphenyl, fluorenyl, dibenzofuranyl, dibenzothiophenyl, carbazolyl; even further more preferably R³ represents phenyl, m-biphenyl, p-biphenyl, 1-naphthyl, 2-naphthyl, 1-fluorenyl, 2-fluorenyl, 3-fluorenyl, 4-fluorenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl, 2-carbazolyl, 3-carbazolyl; most preferably R³ represents phenyl, m-biphenyl, p-biphenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl; further most preferably R³ represents phenyl;
Y₁, Y₂, and Y₃ each independently represent a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms; a substituted or unsubstituted aromatic heterocyclic group which has a ring formed of 3 to 24 atoms or CN; preferably, Y₁, Y₂, and Y₃ each independently represent a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, a substituted or unsubstituted aromatic heterocyclic group which has a ring formed of 3 to 18 atoms or CN; more preferably, Y₁, Y₂, and Y₃ each independently represent a substituted or unsubstituted alkyl group having 1 to 4 carbon atoms, a substituted or unsubstituted cycloalkyl group having a ring formed of 5 or 6 carbon atoms, alkoxy group having 1 to 4 carbon atoms, phenyl, biphenyl, terphenyl, naphthyl, phenanthrenyl, dibenzofuranyl, dibenzothiophenyl, carbazolyl, or CN;
d and f each represent a number of 0, 1, 2, or 3, preferably 0, 1 or 2, more preferably 0 or 1, most preferably 0; and e represents a number of 0, 1, or 2, preferably 0 or 1, more preferably 0.

Preferably, the ring A, the ring B, the ring C and the ring D in the group of formula (VI) or (VII) in the group A₂ each independently represent 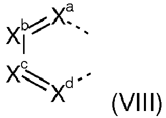 wherein
X^{a} is CR^{a} or N, preferably CR^{a},
X^{b} is CR^{b} or N, preferably CR^{b},
X^{c} is CR^{c} or N, preferably CR^{c},
X^{d} is CR^{d} or N, preferably CR^{d},
or 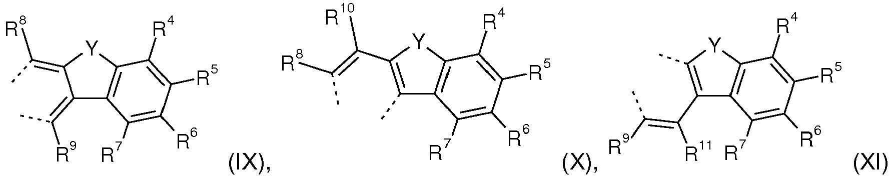 wherein
Y is NR¹², CR¹³R¹⁴, O or S, preferably N-Ph, C(CH₃)₂, O or S,
R^{a}, R^{b}, R^{c}, R^{d}, R⁴, R⁵, R⁶, R⁷, R⁸, R⁹, R¹⁰ and R¹¹ each independently represent hydrogen, CN, a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; preferably R^{a}, R^{b}, R^{c}, R^{d}, R⁴, R⁵, R⁶, R⁷, R⁸, R⁹, R¹⁰ and R¹¹ each independently represent hydrogen, CN, a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R^{a}, R^{b}, R^{c}, R^{d}, R⁴, R⁵, R⁶, R⁷, R⁸, R⁹, R¹⁰ and R¹¹ each independently represent hydrogen, CN, a substituted or unsubstituted alkyl group having 1 to 4 carbon atoms, a substituted or unsubstituted cycloalkyl group having a ring formed of 5 or 6 carbon atoms, alkoxy group having 1 to 4 carbon atoms, phenyl, biphenyl, terphenyl, naphthyl, phenanthrenyl, fluorenyl, dibenzofuranyl, dibenzothiophenyl, carbazolyl; most preferably, R^{a}, R^{b}, R^{c}, R^{d}, R⁴, R⁵, R⁶, R⁷, R⁸, R⁹, R¹⁰ and R¹¹ each represent hydrogen or one or two, preferably one, of R^{a}, R^{b}, R^{c}, R^{d}, R⁴, R⁵, R⁶, R⁷, R⁸, R⁹, R¹⁰ and R¹¹ represent phenyl and the remaining of R^{a}, R^{b}, R^{c}, R^{d}, R⁴, R⁵, R⁶, R⁷, R⁸, R⁹, R¹⁰ and R¹¹ represent hydrogen;
   or
two adjacent groups R^{a}, R^{b}, R^{c}, R^{d}, R⁴, R⁵, R⁶, R⁷, R⁸, R⁹, R¹⁰ and R¹¹ may form together a carbocyclic or heterocyclic ring which may be unsubstituted or substituted; preferably the carbocyclic ring is phenyl, naphtyl or triphenyl and the heterocyclic ring is pyridine, most preferably, the carbocyclic or heterocyclic ring phenyl;
R¹², R¹³ and R¹⁴ each independently represent a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; preferably R¹², R¹³ and R¹⁴ each independently represent a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R¹², R¹³ and R¹⁴ each independently represent a substituted or unsubstituted alkyl group having 1 to 4 carbon atoms, a substituted or unsubstituted cycloalkyl group having a ring formed of 5 or 6 carbon atoms, alkoxy group having 1 to 4 carbon atoms, phenyl, biphenyl, terphenyl, naphthyl, phenanthrenyl, fluorenyl, dibenzofuranyl, dibenzothiophenyl, carbazolyl; most preferably, R¹², R¹³ and R¹⁴ each independently represent methyl, ethyl, hexyl or phenyl;
wherein in one of the rings C or D in formula (VII) one of R^{a}, R^{b}, R^{c} or R^{d}, R⁴, R⁵, R⁶ or R⁷ is a bonding site, wherein in the case that one of R^{a}, R^{b}, R^{c} or R^{d} is a bonding site, at least one of the groups X^{a}, X^{b}, X^{c} and X^{d} is CR^{a}, CR^{b}, CR^{c} or CR^{d}; and
wherein the dotted lines are bonding sites.

Preferably, A₂ represents in the polycyclic compounds of formula (I) or (II) according to the present invention a group of formula 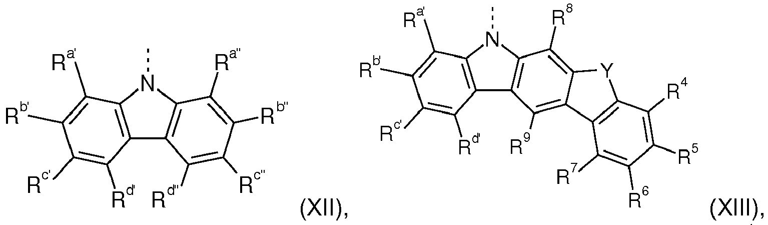 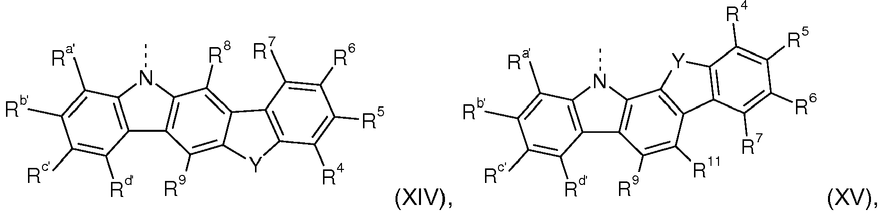 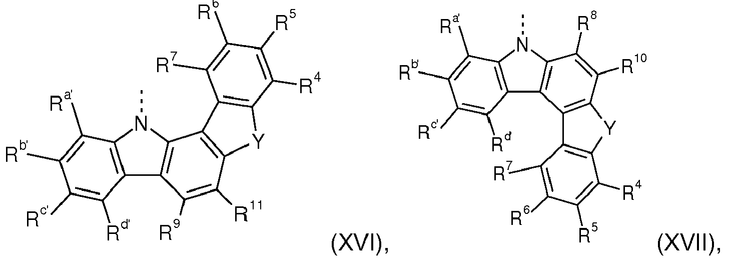 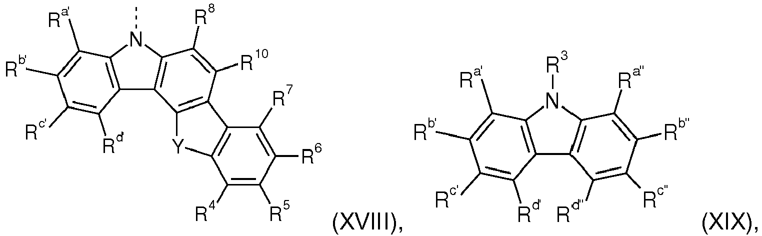 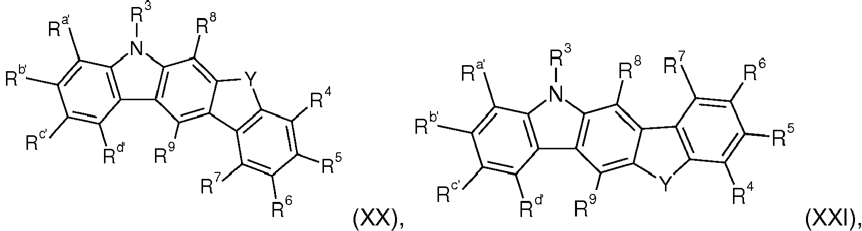 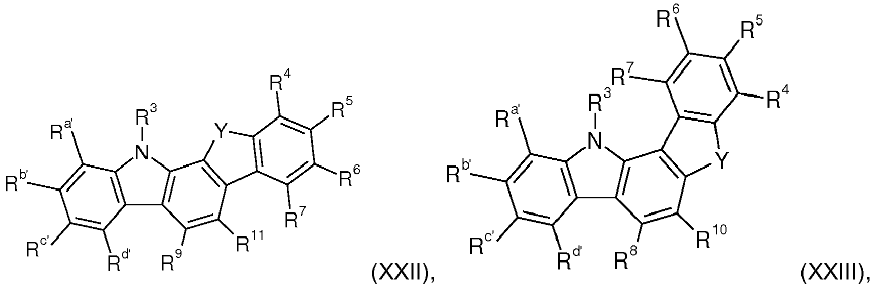 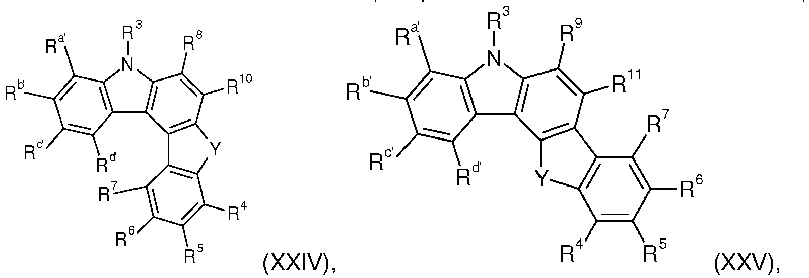 wherein
Y is NR¹², CR¹³R¹⁴, O or S, preferably N-Ph; C(CH₃)₂, O or S,
R^{a'}, R^{b'}, R^{c'}, R^{d'}, R^{a"}, R^{b"}, R^{c"}, R^{d"}, R⁴, R⁵, R⁶, R⁷, R⁸, R⁹, R¹⁰ and R¹¹ each independently represent hydrogen, CN, a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; preferably R^{a'}, R^{b'}, R^{c'}, R^{d'}, R^{a"}, R^{b"}, R^{c"}, R^{d"}, R⁴, R⁵, R⁶, R⁷, R⁸, R⁹, R¹⁰ and R¹¹ each independently represent hydrogen, CN, a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R^{a'}, R^{b'}, R^{c'}, R^{d'}, R^{a"}, R^{b"}, R^{c"}, R^{d"}, R⁴, R⁵, R⁶, R⁷, R⁸, R⁹, R¹⁰ and R¹¹ each independently represent hydrogen, CN, a substituted or unsubstituted alkyl group having 1 to 4 carbon atoms, a substituted or unsubstituted cycloalkyl group having a ring formed of 5 or 6 carbon atoms, alkoxy group having 1 to 4 carbon atoms, phenyl, biphenyl, terphenyl, naphthyl, phenylthrenyl, fluorenyl, dibenzofuranyl, dibenzothiophenyl, carbazolyl; most preferably, R^{a'}, R^{b'}, R^{c'}, R^{d'}, R^{a"}, R^{b"}, R^{c"}, R^{d"}, R⁴, R⁵, R⁶, R⁷, R⁸, R⁹, R¹⁰ and R¹¹ each represent hydrogen or one or two, preferably one, of R^{a'}, R^{b'}, R^{c'}, R^{d'}, R^{a"}, R^{b"}, R^{c"}, R^{d"}, R⁴, R⁵, R⁶, R⁷, R⁸, R⁹, R¹⁰ and R¹¹ represent phenyl and the remaining of R^{a'}, R^{b'}, R^{c'}, R^{d'}, R^{a"}, R^{b"}, R^{c"}, R^{d"}, R⁴, R⁵, R⁶, R⁷, R⁸, R⁹, R¹⁰ and R¹¹ represent hydrogen;
   or
two adjacent groups R^{a'}, R^{b'}, R^{c'}, R^{d'}, R^{a"}, R^{b"}, R^{c"}, R^{d"}, R⁴, R⁵, R⁶, R⁷, R⁸, R⁹, R¹⁰ and R¹¹ may form together a carbocyclic or heterocyclic ring which may be unsubstituted or substituted;
R¹², R¹³ and R¹⁴ each independently represent a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; preferably R¹², R¹³ and R¹⁴ each independently represent a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R¹², R¹³ and R¹⁴ each independently represent a substituted or unsubstituted alkyl group having 1 to 4 carbon atoms, a substituted or unsubstituted cycloalkyl group having a ring formed of 5 or 6 carbon atoms, alkoxy group having 1 to 4 carbon atoms, phenyl, biphenyl, terphenyl, naphthyl, phenylthrenyl, fluorenyl, dibenzofuranyl, dibenzothiophenyl, carbazolyl; most preferably, R¹², R¹³ and R¹⁴ each independently represent methyl, ethyl, hexyl or phenyl;
wherein one of R^{a'}, R^{b'}, R^{c'}, R^{d'}, R^{a"}, R^{b"}, R^{c"} and R^{d"} in formula (XIX) is a bonding site, wherein one of R^{a'}, R^{b'}, R^{c'}, R^{d'}, R⁴, R⁵, R⁶ and R⁷, preferably one of R^{a'}, R^{b'}, R^{c'}, R^{d'} in formulae (XX), (XXI), (XXII), (XXIII), (XXIV) and (XXV) is a bonding site; and
R³ represents a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms, preferably, R³ in the group of formula (V) represents a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R³ represents a substituted or unsubstituted aromatic hydrocarbon group having a ring formed of 6 to 18 carbon atoms, or a substituted or unsubstituted aromatic heterocyclic group which has a ring formed of 3 to 18 atoms, further more preferably R³ represents phenyl, biphenyl, terphenyl, naphthyl, phenanthrenyl, triphenyl, fluorenyl, dibenzofuranyl, dibenzothiophenyl, carbazolyl; even further more preferably R³ represents phenyl, m-biphenyl, p-biphenyl, 1-naphthyl, 2-naphthyl,
1-fluorenyl, 2-fluorenyl, 3-fluorenyl, 4-fluorenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl, 2-carbazolyl, 3-carbazolyl; most preferably R³ represents phenyl, m-biphenyl, p-biphenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl; further most preferably R³ represents phenyl.

In the polycyclic compounds (I) and (II) according to the present invention d and f each represent a number of 0, 1, 2, or 3, preferably 0, 1 or 2, more preferably 0 or 1, most preferably 0; and e represents a number of 0, 1, or 2, preferably 0 or 1, more preferably 0. Preferably, the polycyclic compounds (I) and (II) each comprise a total number of substituents represented by Y¹, Y² and Y³ of 3 or less, i.e. the sum of d, e and f is 0, 1, 2 or 3, preferably 0, 1 or 2, more preferably 0 or 1, most preferably 0.

L₁ in the polycyclic compounds (I) and (II) according to the present invention most preferably represents a single bond or a phenylene group and further most preferably a single bond. Preferred phenylene groups are 1,4-phenylene, 1,3-phenylene and 1,2-phenylene.

L₂ in the polycyclic compounds (I) and (II) according to the present invention most preferably represents a single bond.

Preferred polycyclic compounds (I) and (II) according to the present invention having one of the following formulae: 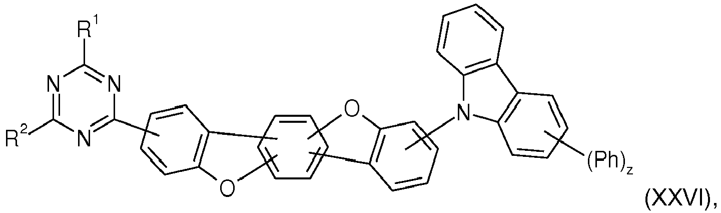 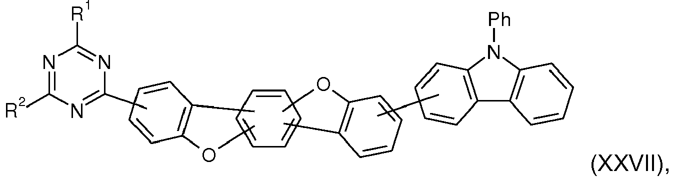 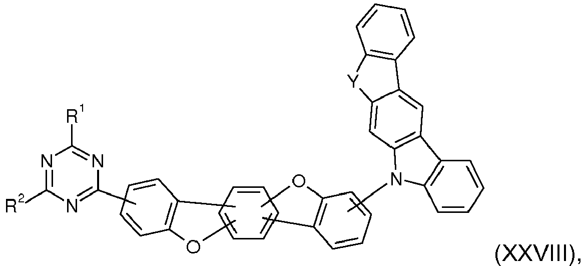 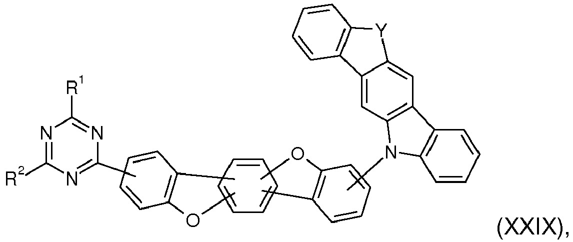 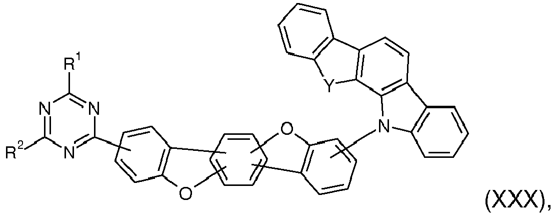 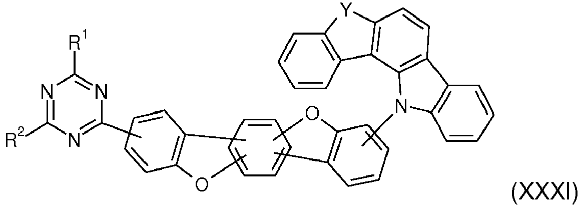 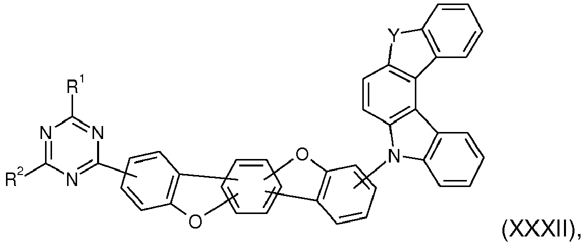 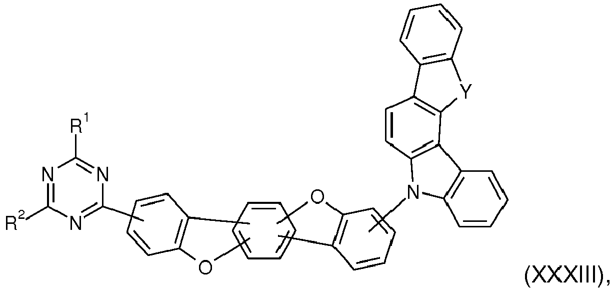 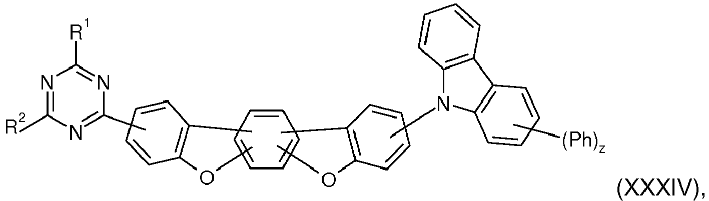 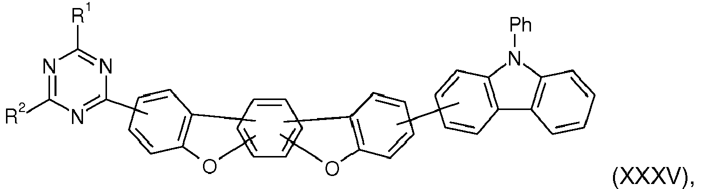 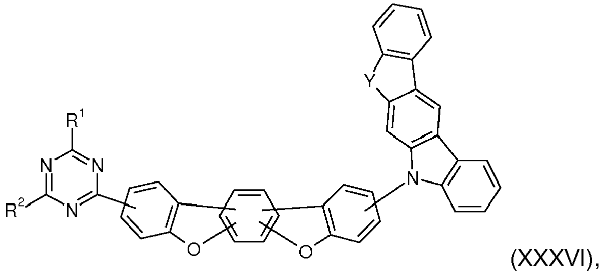 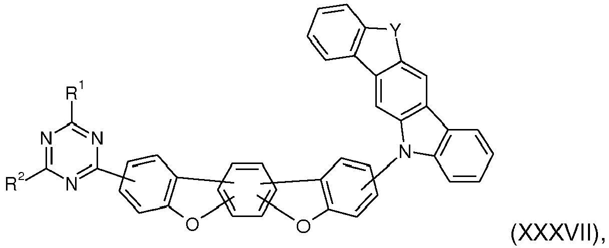 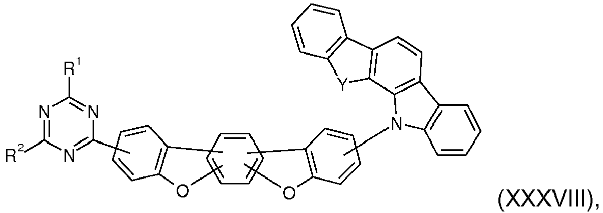 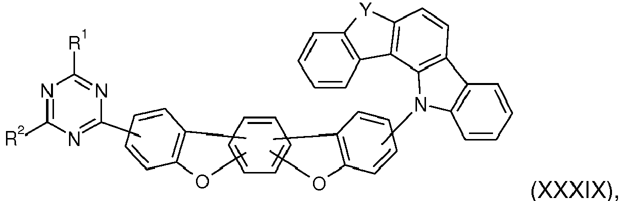 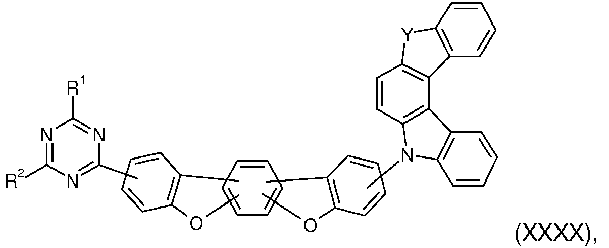 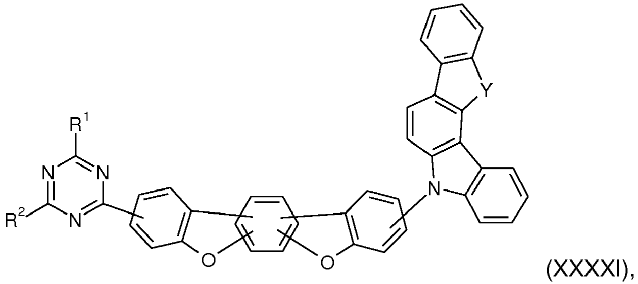 wherein
Y is NR¹², CR¹³R¹⁴, O or S, preferably N-Ph, C(CH₃)₂, O or S, and z is 0 or 1.
R¹², R¹³ and R¹⁴ each independently represent a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; preferably R¹², R¹³ and R¹⁴ each independently represent a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R¹², R¹³ and R¹⁴ each independently represent a substituted or unsubstituted alkyl group having 1 to 4 carbon atoms, a substituted or unsubstituted cycloalkyl group having a ring formed of 5 or 6 carbon atoms, alkoxy group having 1 to 4 carbon atoms, phenyl, biphenyl, terphenyl, naphthyl, phenanthrenyl, fluorenyl, dibenzofuranyl, dibenzothiophenyl, carbazolyl; most preferably, R¹², R¹³ and R¹⁴ each independently represent methyl, ethyl, hexyl or phenyl;
R¹ and R² each independently represent hydrogen a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; preferably, R¹ and R² in the group of formula (V) each independently represent a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R¹ and R² each independently represent a substituted or unsubstituted aromatic hydrocarbon group having a ring formed of 6 to 18 carbon atoms, or a substituted or unsubstituted aromatic heterocyclic group which has a ring formed of 3 to 18 atoms, further more preferably R¹ and R² each independently represent phenyl, biphenyl, terphenyl, naphthyl, phenanthrenyl, fluorenyl, dibenzofuranyl, dibenzothiophenyl, carbazolyl; even further more preferably R¹ and R² each independently represent phenyl, m-biphenyl, p-biphenyl, 1-naphthyl, 2-naphthyl, 1-fluorenyl, 2-fluorenyl, 3-fluorenyl, 4-fluorenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl, 2-carbazolyl, 3-carbazolyl; most preferably R¹ and R² each independently represent phenyl, m-biphenyl, p-biphenyl, 1-fluorenyl, 2-fluorenyl, 3-fluorenyl, 4-fluorenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl; further most preferably R¹ and R² each represent phenyl;
preferably 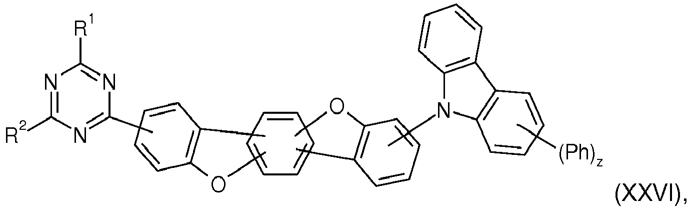 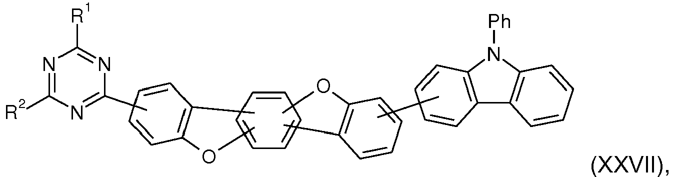 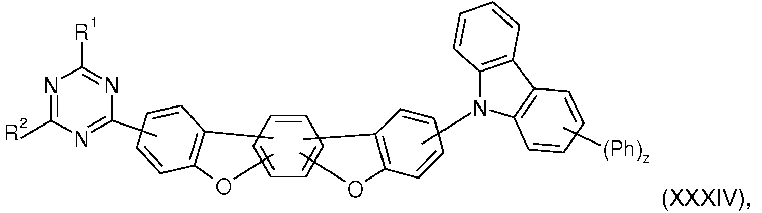 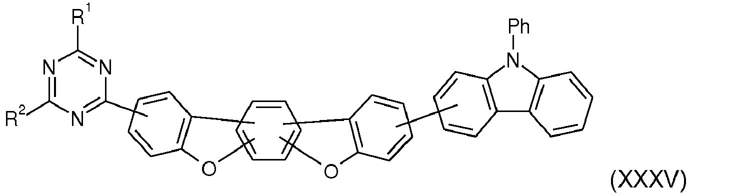 wherein
R¹ and R² each independently represent hydrogen a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; preferably, R¹ and R² in the group of formula (V) each independently represent a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; more preferably, R¹ and R² each independently represent a substituted or unsubstituted aromatic hydrocarbon group having a ring formed of 6 to 18 carbon atoms, or a substituted or unsubstituted aromatic heterocyclic group which has a ring formed of 3 to 18 atoms, further more preferably R¹ and R² each independently represent phenyl, biphenyl, terphenyl, naphthyl, phenanthrenyl, fluorenyl, dibenzofuranyl, dibenzothiophenyl, carbazolyl; even further more preferably R¹ and R² each independently represent phenyl, m-biphenyl, p-biphenyl, 1-naphthyl, 2-naphthyl, 1-fluorenyl, 2-fluorenyl, 3-fluorenyl, 4-fluorenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl, 2-carbazolyl, 3-carbazolyl; most preferably R¹ and R² each independently represent phenyl, m-biphenyl, p-biphenyl, 1-fluorenyl, 2-fluorenyl, 3-fluorenyl, 4-fluorenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl; further most preferably R¹ and R² each represent phenyl; and
z is 0 or 1.

Further, the present invention provides an organic electroluminescence (EL) device comprising a cathode, an anode, and a plurality of organic layers provided between the cathode and the anode, wherein at least one of the plurality of organic layers is an emitting layer, wherein at least one organic layer comprises at least one polycyclic compound of formula (I) or (II) according to the present invention.

The invention further relates to the use of a polycyclic compound of formula (I) or (II) according to the present invention in an organic electroluminescence device.

The present invention provides a novel material useful as a material for organic EL devices and an organic EL device comprising the material. The compounds of formulae (I) and (II) are suitable as material for electronic devices, preferably OLEDs, especially as a host material, a charge transporting material, charge and/or exciton blocking material, preferably as a host material and/or a charge transporting material, more preferably as a host material, having a good overall performance, especially improved efficiency and/or lifetime.

FIG. 1 is a schematic illustration showing an example of the structure of an organic electroluminescence device according to an embodiment of the invention.

The term of "XX to YY carbon atoms" referred to by "a substituted or unsubstituted group ZZ having XX to YY carbon atoms" used herein is the number of carbon atoms of the unsubstituted group ZZ and does not include any carbon atom in the substituent of the substituted group ZZ. "YY" is larger than "XX" and each of "XX" and "YY" represents an integer of 1 or more.

The term of "XX to YY atoms" referred to by "a substituted or unsubstituted group ZZ having XX to YY atoms" used herein is the number of atoms of the unsubstituted group ZZ and does not include any atom in the substituent of the substituted group ZZ. "YY" is larger than "XX" and each of "XX" and "YY" represents an integer of 1 or more.

The term "C_{ZZ}-C_{YY}-" referred to by "C_{ZZ}-C_{YY}-ZZ group which is unsubstituted or substituted" used herein is the number of carbon atoms of the unsubstituted group ZZ and does not include any heteroatom or any atom in the substituent of the substituted group ZZ. "YY" is larger than "XX" and each of "XX" and "YY" represents an integer of 1 or more.

The term of "unsubstituted group ZZ" referred to by "a substituted or unsubstituted group ZZ" used herein means the group ZZ wherein no hydrogen atom is substituted by a substituent.

The number of "ring carbon atoms" ("a ring formed of carbon atoms") referred to herein means the number of the carbon atoms included in the atoms which are members forming the ring itself of a compound in which a series of atoms is bonded to form the ring (for example, a monocyclic compound, a fused ring compound, a cross-linked compound, a carbocyclic compound, and a heterocyclic compound). If the ring has a substituent, the carbon atom in the substituent is not included in the ring carbon atom. The same applies to the number of "ring carbon atom" described below, unless otherwise noted. For example, a benzene ring has 6 ring carbon atoms, a naphthalene ring has 10 ring carbon atoms, a pyridinyl group has 5 ring carbon atoms, and a furanyl group has 4 ring carbon atoms. If a benzene ring or a naphthalene ring has, for example, an alkyl substituent, the carbon atom in the alkyl substituent is not counted as the ring carbon atom of the benzene or naphthalene ring. In case of a fluorene ring to which a fluorene substituent is bonded (inclusive of a spirofluorene ring), the carbon atom in the fluorene substituent is not counted as the ring carbon atom of the fluorene ring.

The number of "ring atom" ("a ring formed of atoms") referred to herein means the number of the atoms which are members forming the ring itself (for example, a monocyclic ring, a fused ring, and a ring assembly) of a compound in which a series of atoms is bonded to form the ring (for example, a monocyclic compound, a fused ring compound, a cross-linked compound, a carbocyclic compound, and a heterocyclic compound). The atom not forming the ring (for example, hydrogen atom(s) for saturating the valence of the atom which forms the ring) and the atom in a substituent, if the ring is substituted, are not counted as the ring atom. The same applies to the number of "ring atoms" described below, unless otherwise noted. For example, a pyridine ring has 6 ring atoms, a quinazoline ring has 10 ring atoms, and a furan ring has 5 ring atoms. The hydrogen atom on the ring carbon atom of a pyridine ring or a quinazoline ring and the atom in a substituent are not counted as the ring atom. In case of a fluorene ring to which a fluorene substituent is bonded (inclusive of a spirofluorene ring), the atom in the fluorene substituent is not counted as the ring atom of the fluorene ring.

The definition of "hydrogen atom" used herein includes isotopes different in the neutron numbers, i.e., light hydrogen (protium), heavy hydrogen (deuterium), and tritium.

The residues mentioned in the specification of the present application generally have the following preferred meanings, if said residues are not further specified in specific embodiments mentioned below:
Halogen is generally fluorine, chlorine, bromine and iodine.

Examples of the substituted or unsubstituted aromatic hydrocarbon group having a ring formed of 6 carbon atoms include substituted or unsubstituted benzene. Examples of the substituted or unsubstituted aromatic hydrocarbon group having a ring formed of 6 to 24 carbon atoms include residues having corresponding valencies of substituted or unsubstituted benzene and polycyclic aromatic hydrocarbons like naphthalene, biphenyl, terphenyl, fluorene, phenanthrene, triphenylene, perylene, chrysene, fluoranthene, benzofluorene, benzotriphenylene, benzochrysene, and anthracene. Preferred are benzene, naphthalene, biphenyl, terphenyl, fluorene, and phenanthrene.

Examples of the substituted or unsubstituted aromatic heterocyclic group having a ring formed of 6 atoms include substituted or unsubstituted pyridine, pyridazine, pyrimidine, pyrazine, and 1,3,5 -triazine. Examples of the substituted or unsubstituted aromatic heterocyclic group having a ring formed of 3 to 24 atoms include residues having corresponding valencies of substituted or unsubstituted pyridine, pyridazine, pyrimidine, pyrazine, 1,3,5-triazine, 1,2,4-triazine, carbazole, dibenzofuran, dibenzothiophene, phenoxazine, phenothiazine, and dihydroacridine as well as the following residues thienyl, benzothiophenyl, thianthrenyl, furyl, furfuryl, 2H-pyranyl, benzofuranyl, isobenzofuranyl, phenoxythienyl, pyrrolyl, imidazolyl, pyrazolyl, bipyridyl, pyrimidinyl, pyrazinyl, pyridazinyl, indolizinyl, isoindolyl, indolyl, indazolyl, purinyl, quinolizinyl, quinolyl, isoquinolyl, phthalazinyl, naphthyridinyl, quinoxalinyl, quinazolinyl, quinolinyl, pteridinyl, carbolinyl, benzotriazolyl, benzoxazolyl, phenanthridinyl, acridinyl, phenanthrolinyl, phenazinyl, isothiazolyl, phenothiazinyl, isoxazolyl, and furazanyl. Preferred are pyridine, pyridazine, pyrimidine, pyrazine, carbazole, dibenzofuran, dibenzothiophene, phenoxazine, and dihydroacridine.

Examples of the alkyl group, alkylene group, and trivalent or tetravalent saturated hydrocarbon group, each of which has 1 to 20 carbon atoms include a methyl group, an ethyl group, a propyl group, an isopropyl group, a n-butyl group, a s-butyl group, a t-butyl group, an isobutyl group, a n-pentyl group, a n-hexyl group, a n-heptyl group, a n-octyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, a n-octadecyl group, a neopentyl group, a 1-methylpentyl group, a 2-methylpentyl group, a 1-pentylhexyl group, a 1-butylpentyl group, a 1-heptyloctyl group, and a 3-methylpentyl group, and groups obtained by allowing those groups to have two to four valencies. Preferred are a methyl group, an ethyl group, a propyl group, an isopropyl group, a n-butyl group, a s-butyl group, a isobutyl group, a t-butyl group, a n-pentyl group, a n-hexyl group, a n-heptyl group, a n-octyl group, a n-nonyl group, a n-decyl group, a n-undecyl group, a n-dodecyl group, a n-tridecyl group, a n-tetradecyl group, a n-pentadecyl group, a n-hexadecyl group, a n-heptadecyl group, a n-octadecyl group, a neopentyl group, a 1-methylpentyl group, a 1-pentylhexyl group, a 1-butylpentyl group, and a 1-heptyloctyl group.

Examples of the substituted or unsubstituted cycloalkyl group, cycloalkylene group, and trivalent or tetravalent cyclic saturated hydrocarbon group, each of which has a ring formed of 3 to 20 carbon atoms, include a cyclopropyl group, a cyclobutyl group, a cyclopentyl group, and a cyclohexyl group, and groups obtained by allowing those group to have two to four valencies. Preferred are a cyclobutyl group, a cyclopentyl group, and a cyclohexyl group.

Examples of the alkoxy group having 1 to 20 carbon atoms include a methoxy group, an ethoxy group, a methoxy group, an i-propoxy group, a n-propoxy group, a n-butoxy group, a s-butoxy group, and a t-butoxy group. Preferred are a methoxy group, an ethoxy group, an i-propoxy group, and a n-propoxy group.

Examples of the substituted silyl group having 1 to 20 carbon atoms include a trimethylsilyl group, a triethylsilyl group, a tributylsilyl group, a trioctylsilyl group, a triisobutylsilyl group, a dimethylethylsilyl group, a dimethylisopropylsilyl group, a dimethylpropylsilyl group, a dimethylbutylsilyl group, a dimethyltert.-butylsilyl group, a diethylisopropylsilyl group, a phenyldimethylsilyl group, a diphenylmethylsilyl group, a biphenyltert.-butylsilyl group, and a triphenylsilyl group, and groups obtained by allowing those groups to have two or three valencies. Preferred are a trimethylsilyl group, a triethylsilyl group, and a tributylsilyl group.

Examples of the aralkyl group having 7 to 24 carbon atoms include a benzyl group, a phenethyl group, and a phenylpropyl group.

Examples of the optional substituents that may substitute each of the group in the formulae (I), (II), (Iz) and (IIz) and the preferred embodiments of the inventive polycyclic compounds mentioned above include alkyl groups each having 1 to 10 carbon atoms (such as a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, a iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, and a 1,2,3-trinitropropyl group), cycloalkyl groups each having a ring formed of 3 to 40 carbon atoms (such as a cyclopropyl group, a cyclobutyl group, a cyclopentyl group, a cyclohexyl group, a 4 -methylcyclohexyl group, a 1-adamantyl group, a 2-adamantyl group, a 1-norbornyl group, and a 2-norbornyl group), alkoxy groups each having 1 to 6 carbon atoms (such as an ethoxy group, a methoxy group, an i-propoxy group, a n-propoxy group, a s-butoxy group, a t-butoxy group, a pentoxy group, and a hexyloxy group), cycloalkoxy groups each having a ring formed of 3 to 10 carbon atoms (such as a cyclopentoxy group and a cyclohexyloxy group), aromatic hydrocarbon groups each having a ring formed of 6 to 40 carbon atoms, aromatic heterocyclic groups each having a ring formed of 3 to 40 atoms, amino groups each substituted with an aromatic hydrocarbon group having a ring formed of 6 to 40 carbon atoms, ester groups each having an aromatic hydrocarbon group having a ring formed of 6 to 40 carbon atoms, an ester group, cyano group, and nitro group, each of which has an alkyl group having 1 to 6 carbon atoms, and halogen atom.

Of those, an alkyl group having 1 to 6 carbon atoms, a phenyl group, a pyridyl group, a carbazolyl group, and a dibenzofuranyl group, a fluorenyl group are preferred.

The optional substituent may be further substituted with an optional substituent mentioned above.

The number of the optional substituents depends on the group which is substituted by said substituent(s). Preferred are 1, 2, 3 or 4 optional substituents, more preferred are 1, 2 or 3 optional substituents, most preferred are 1 or 2 optional substituents. In a further preferred embodiment, the groups mentioned above are unsubstituted.

In the inventive case where A₁ in the polycyclic compounds of the formulae (I) and (II) represents a triazinyl group according to formula (V) and A₂ represents a carbazolyl group according to formula (VI) or (VII), a good overall performance, especially improved efficiency and/or lifetime of the device using the polycyclic compounds of the formulae (I) and (II) according to the present invention is achieved.

### Preparation of the polycyclic compounds of the formulae (I) and (II)

The polycyclic compounds of formulae (I) and (II) according to the present invention are prepared in analogy to preparation processes known in the art, as for example described in EP 2 301 926 A1.

Preferably, the polycyclic compounds of formulae (I) and (II) are prepared by coupling the corresponding halogenated compounds or compounds with an aldehyde functionality with suitable groups for obtaining the polycyclic compounds of formulae (I) and (II) by known methods. Known methods are for halogenated compounds are for example the Suzuki coupling, Heck coupling, Stille coupling.

Known methods for triazine synthesis from an aldehyde is the condensation reaction with a benzamidine followed by oxidation.

The halogenated compounds preferably have the following formulae: 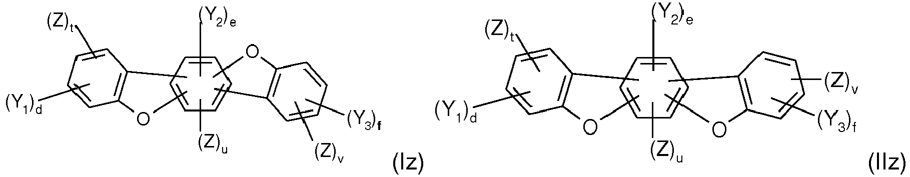 wherein:
the groups and residues Y₁, Y₂, Y₃, d, e and f are as defined above in formulae (I) and (II), and
Z represents a halogen atom, preferably a fluorine atom, a chlorine atom, a bromine atom, and
an iodine atom, a triflate, a tosylate, a mesylate or an aldehyde functional group;
t, u and v each represent 0 or 1, provided that t+u+v ≥ 1.

The halogen or the aldehyde compound represented by formula (Iz) or by formula (IIz) can for example be obtained by subjecting an aromatic halogen compound represented by the following formulae (XXXVIa), (XXXVIb), (XXXVIc), (XXXVId), (XXXVIe) or (XXXVIf) to an intramolecular cyclization reaction.

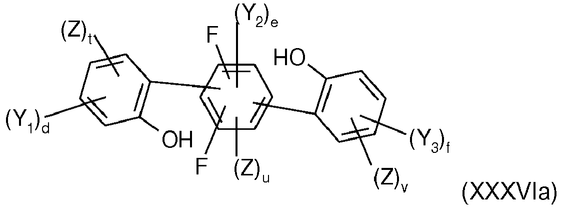

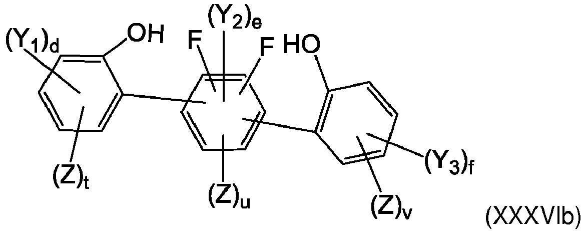

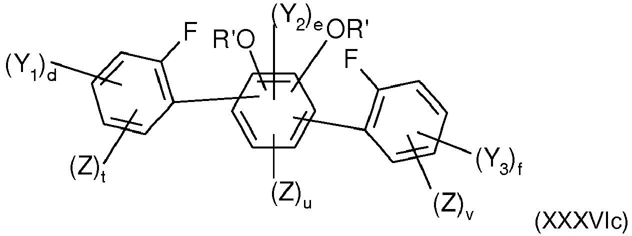

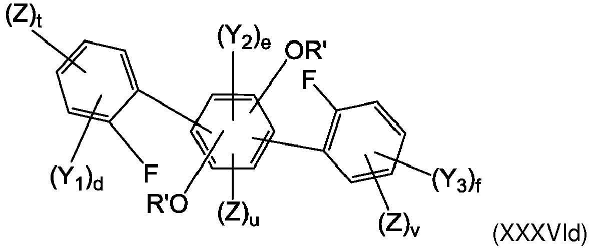

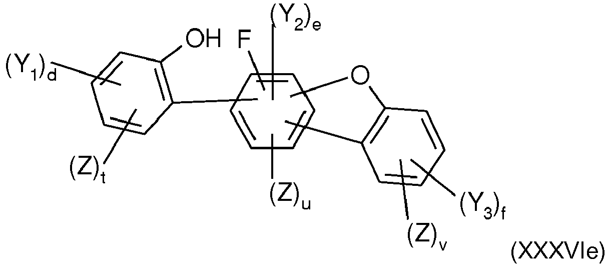

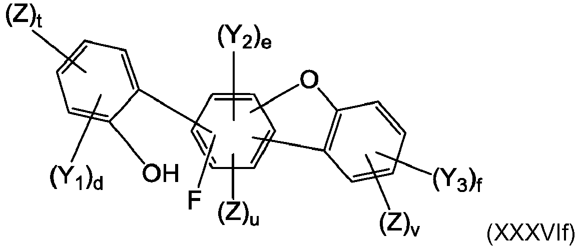

The compounds of formulae (XXXVIa) and (XXXVIb), the compounds of formulae (XXXVIc) and (XXXVId) and the compounds of formulae (XXXVIe) and (XXXVIf) are conformational isomers.

In formulae (XXXVIa), (XXXVIb), (XXXVIc), (XXXVId), (XXXVIe) and (XXXVIf), the groups and residues Y₁, Y₂, Y₃, d, e and f are as defined above in formulae (I) and (II), R' is C₁-C₈alkyl, preferably methyl or ethyl, and
Z represents a halogen atom, preferably a fluorine atom, a chlorine atom, a bromine atom, and an iodine atom or an aldehyde functional group;
t, u and v each represent 0 or 1, provided that t+u+v ≥ 1.

The reaction of the aromatic halogen compound represented by the above-mentioned formulae (XXXVIa), (XXXVIb), (XXXVIc), (XXXVId), (XXXVIe) or (XXXVIf), is typically performed in the presence of a basic catalyst. Examples of the basic catalyst include sodium amide, triethylamine, tributylamine, trioctylamine, pyridine, N,N-dimethylaniline, 1,5-diazabicyclo[4,3,0]nonene-5 (DBN), 1,8-diazabicyclo[5,4,0]undecene-7 (DBU), 1,4-diazabicyclo[2.2.2]octane (DABCO), sodium hydroxide, potassium hydroxide, sodium hydride, sodium phosphate, potassium phosphate, sodium carbonate, potassium carbonate, cesium carbonate, sodium methoxide, sodium ethoxide, sodium t-butoxide and potassium t-butoxide.

The basic catalyst is used for the aromatic halogen compound represented by the formulae (XXXVIa), (XXXVIb), (XXXVIc), (XXXVId), (XXXVIe) or (XXXVIf) as a reaction raw material in such an amount that a molar ratio of the basic catalyst to the aromatic halogen compound is about 1 to 10 and preferably 1 to 5.

A solvent can be used as required at the time of the reaction. Specific examples of the solvent include toluene, xylene, dimethyl formamide (DMF), N,N-dimethylacetamide (DMAc), dimethyl sulfoxide (DMSO), N-methylpyrrolidone (NMP), tetrahydrofuran, dioxane and dimethoxyethane. One kind of them may be used alone, or two or more kinds of them may be used in combination.

The reaction of the aromatic halogen compound represented by the above-mentioned formulae (XXXVIa), (XXXVIb), (XXXVIc), (XXXVId), (XXXVIe) or (XXXVIf) is performed at a temperature of about 0 to 250°C and preferably 100 to 200°C in ordinary cases. When the reaction temperature is 150°C or more, the reaction rate does not reduce but becomes moderate, and hence the reaction time is shortened. The reaction time is typically about 1 minute to 24 hours, and is desirably 1 to 10 hours.

The coupling of the halogen compounds of formulae (Iz) and (IIz) with suitable groups for obtaining the polycyclic compounds of the present invention is known to a person skilled in the art. Suitable examples are given in the example part of the present invention.

In the following two general schemes for the preparation of the compounds of formulae (I) and (II) are shown: 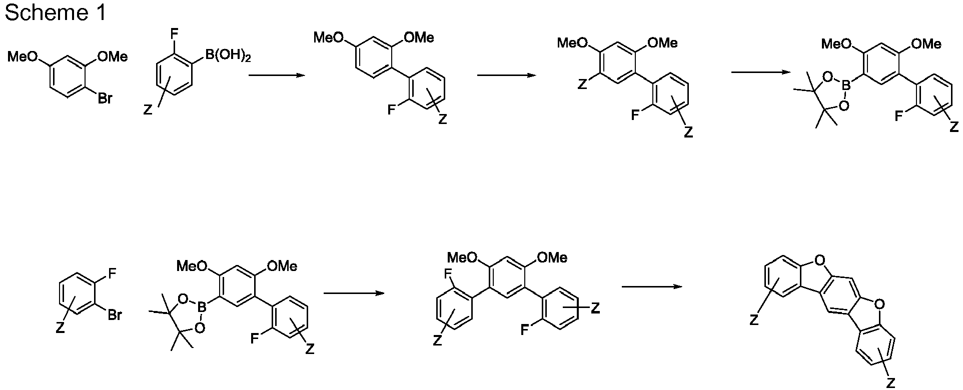 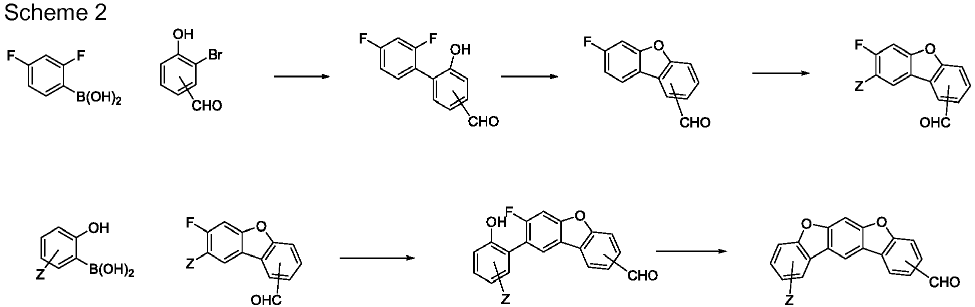 wherein Z represents a halogen atom, preferably a fluorine atom, a chlorine atom, a bromine atom, and an iodine atom, a triflate, a tosylate, a mesylate or an aldehyde functional group; Specific examples of the polycyclic compounds represented by the formulae (I) and (II) of the present invention are shown below. However, the present invention is not limited to these exemplary compounds.

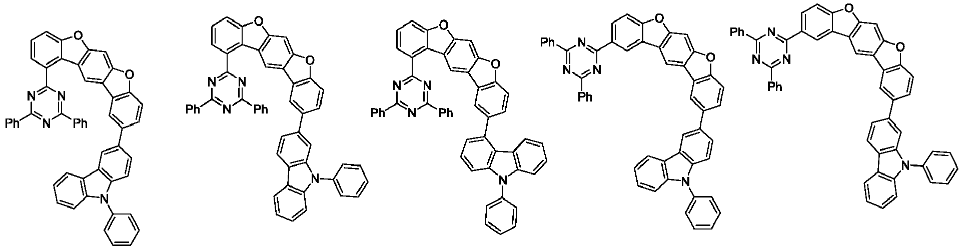

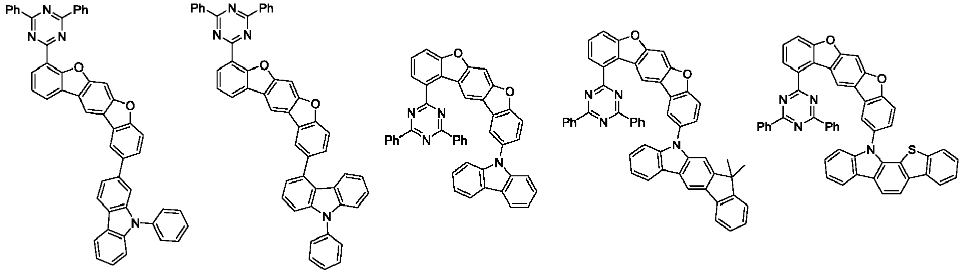

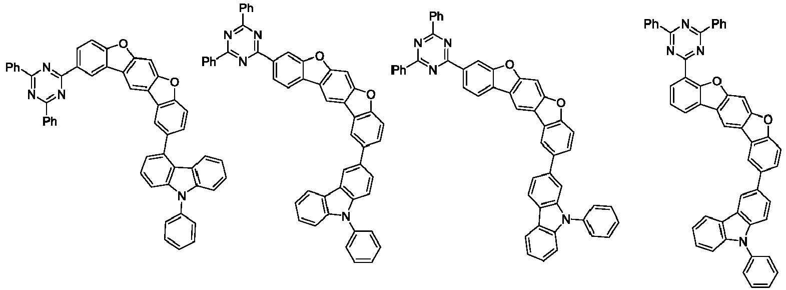

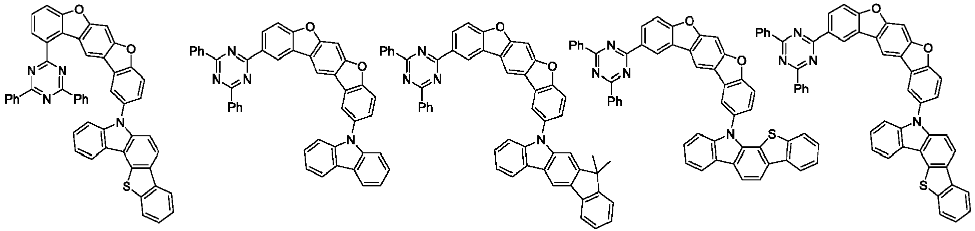

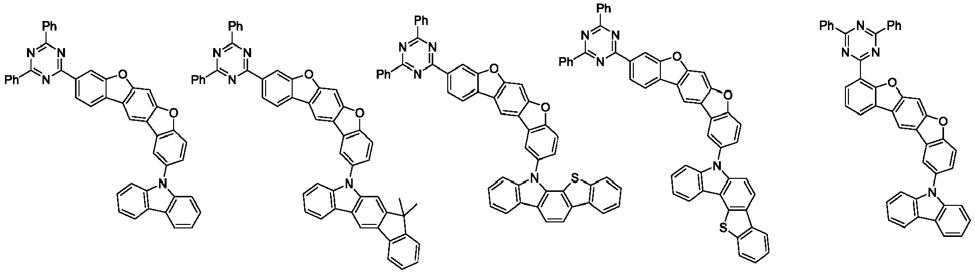

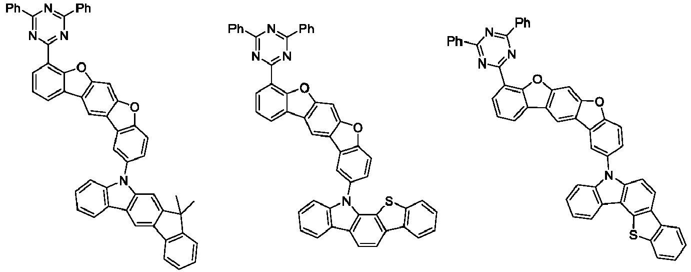

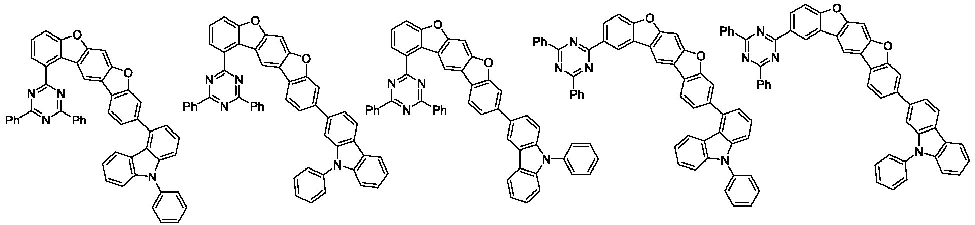

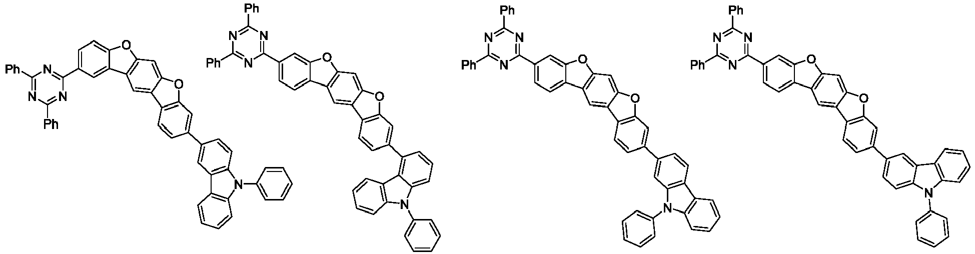

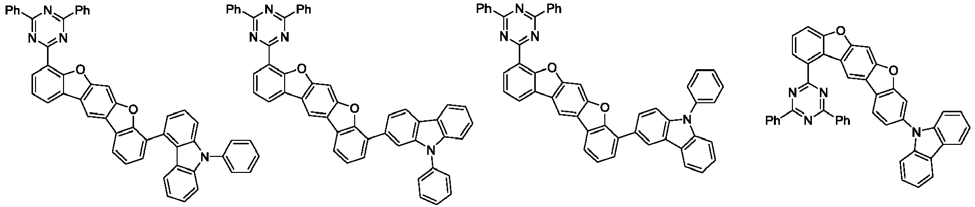

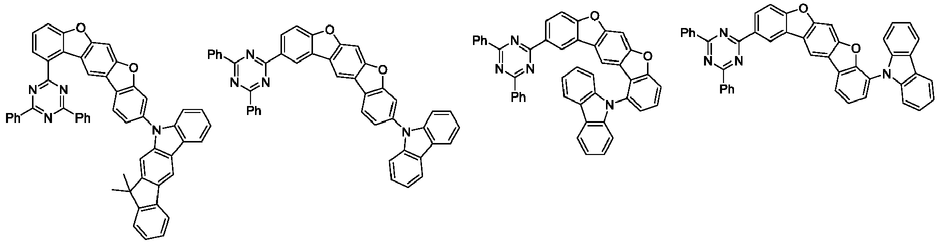

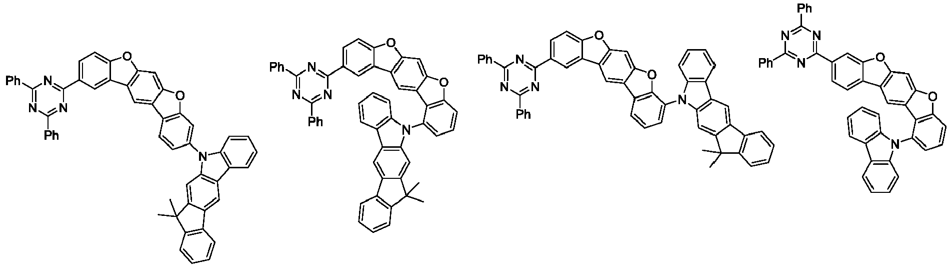

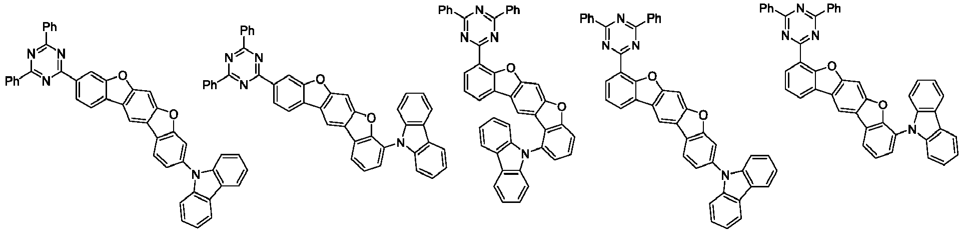

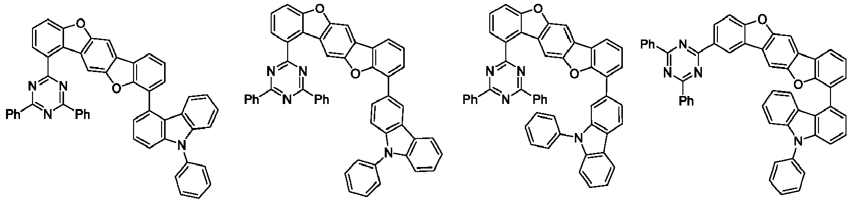

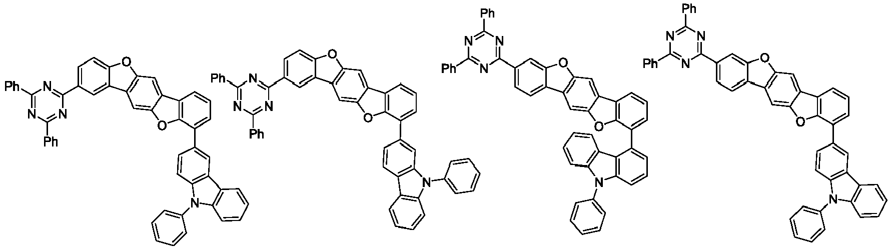

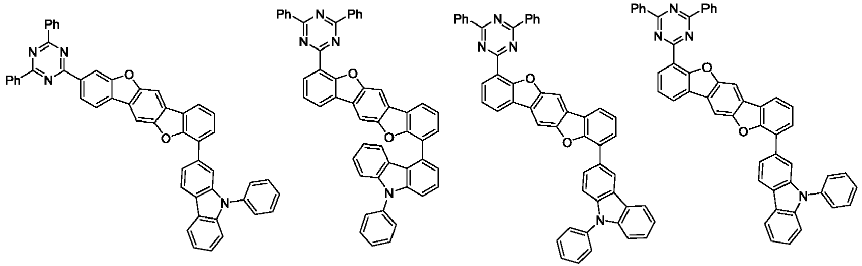

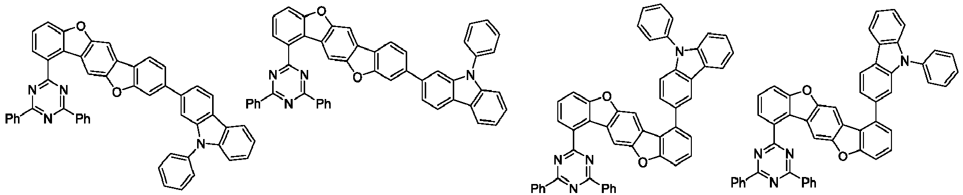

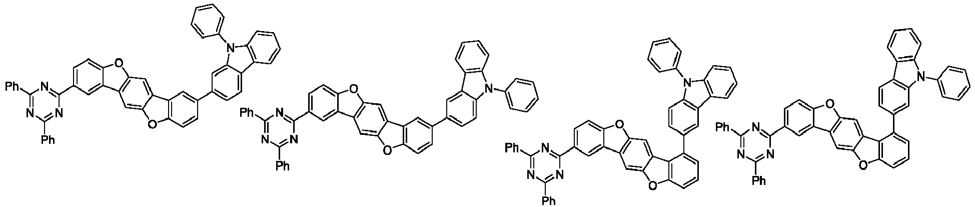

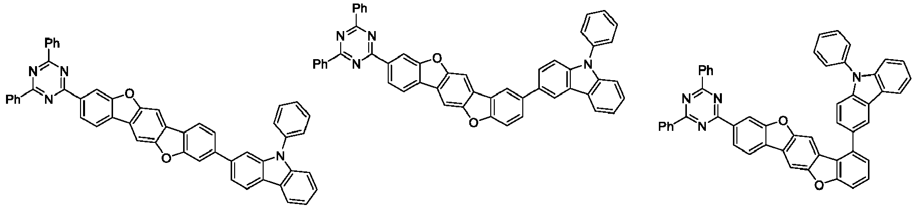

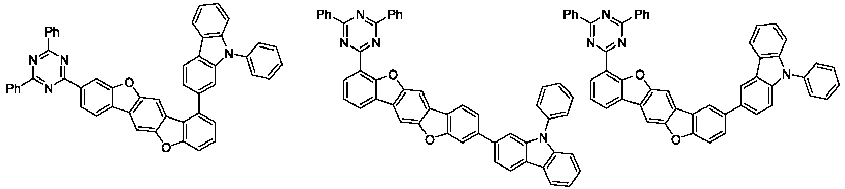

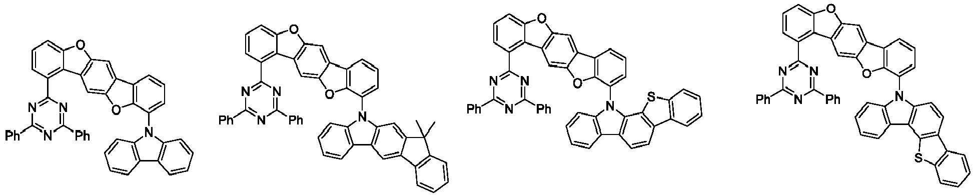

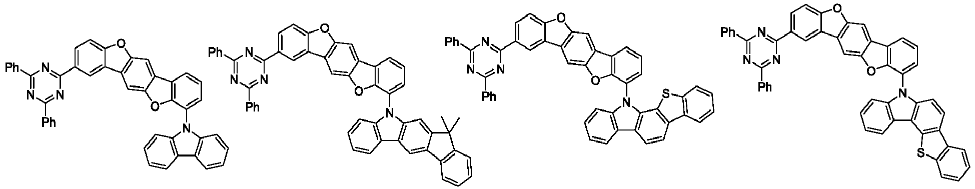

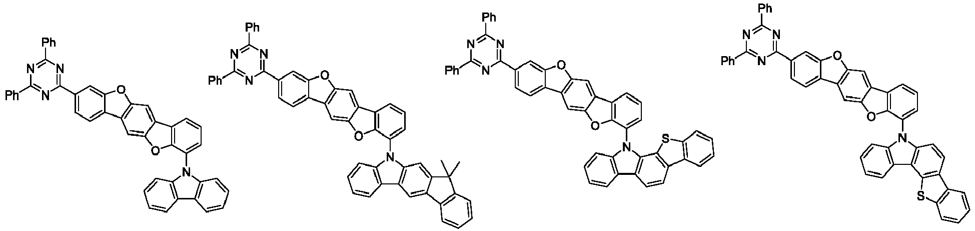

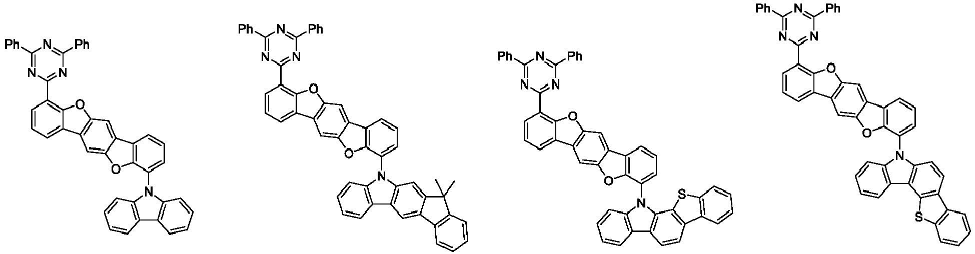

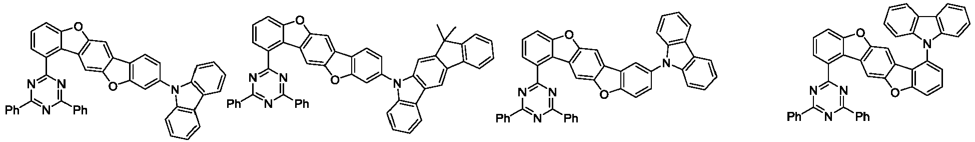

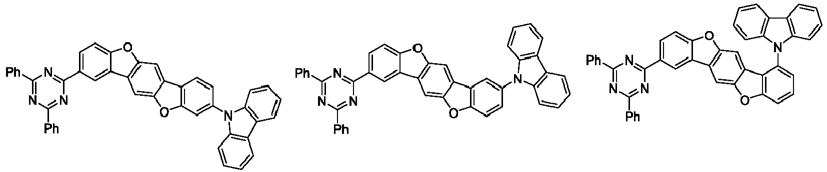

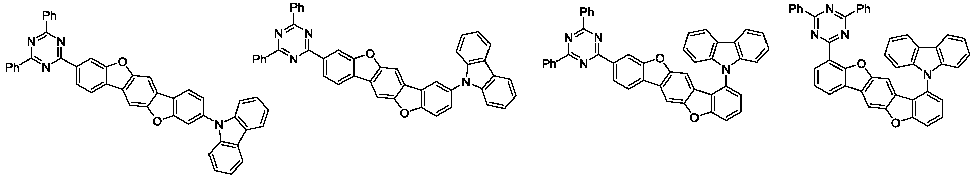

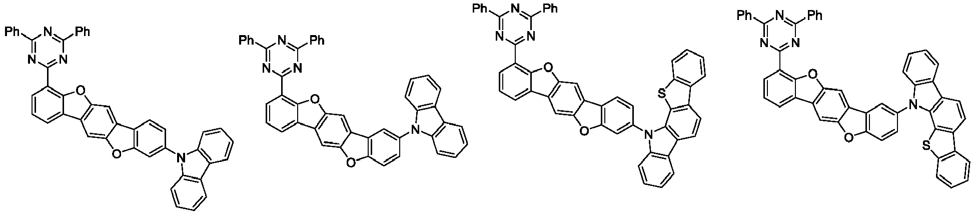

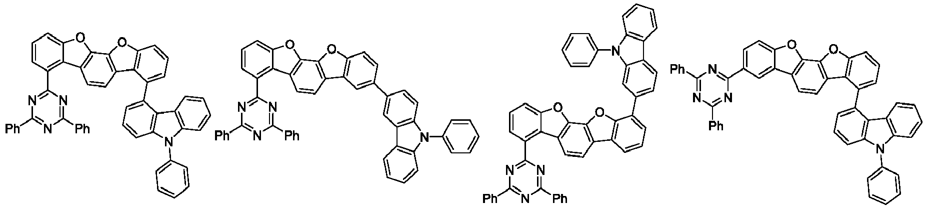

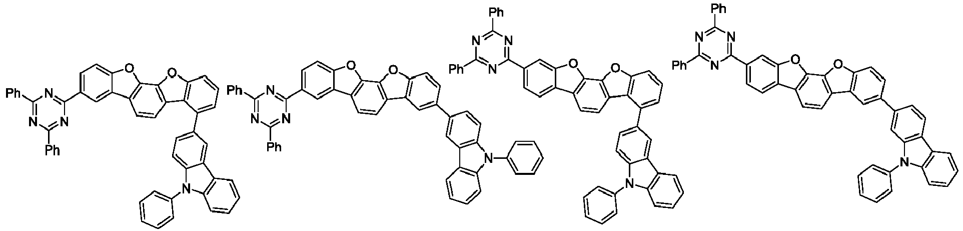

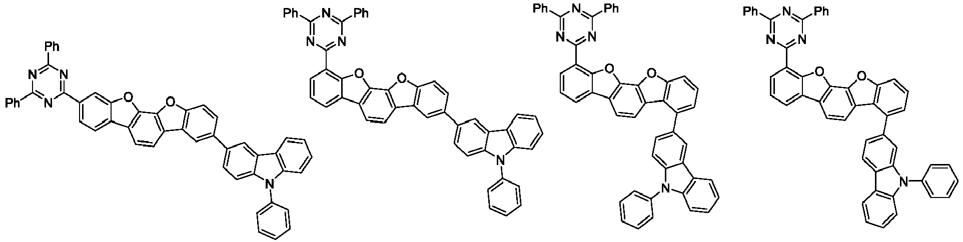

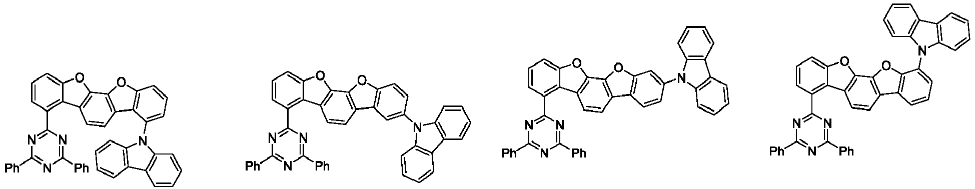

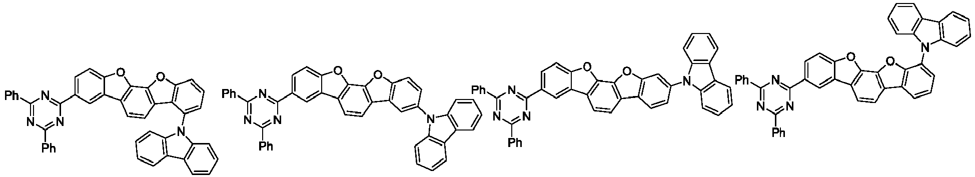

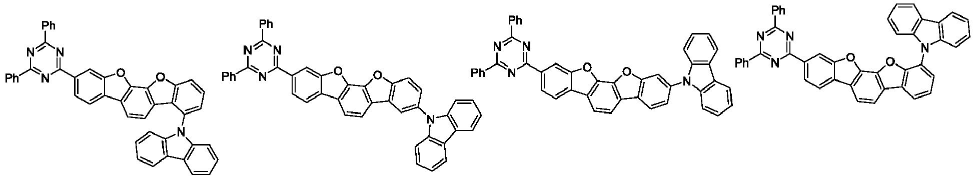

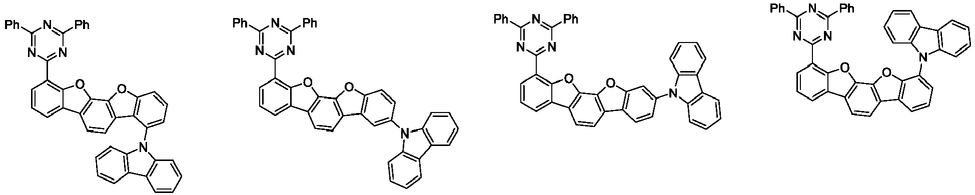

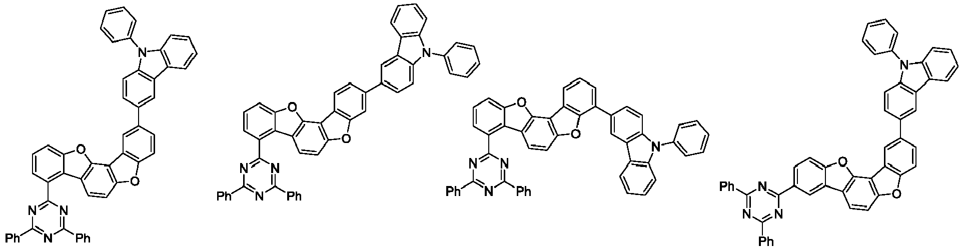

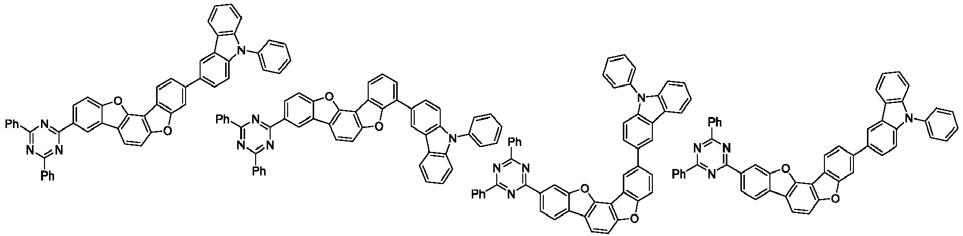

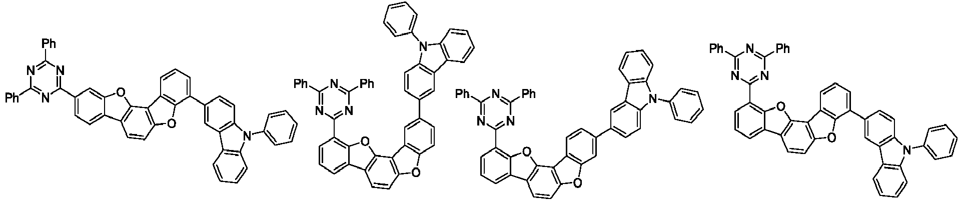

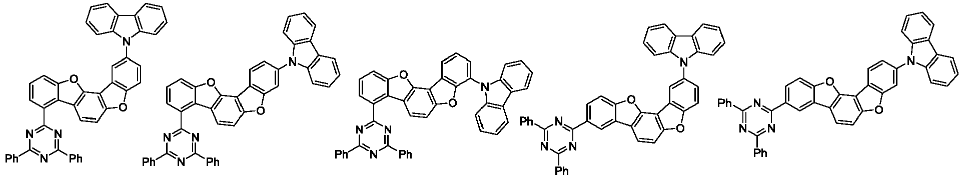

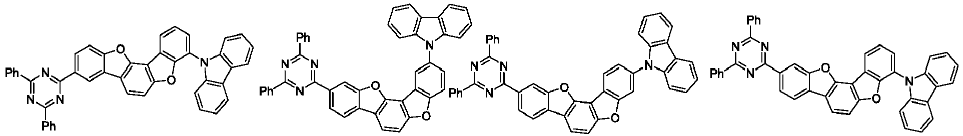

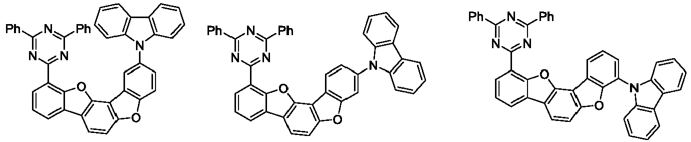

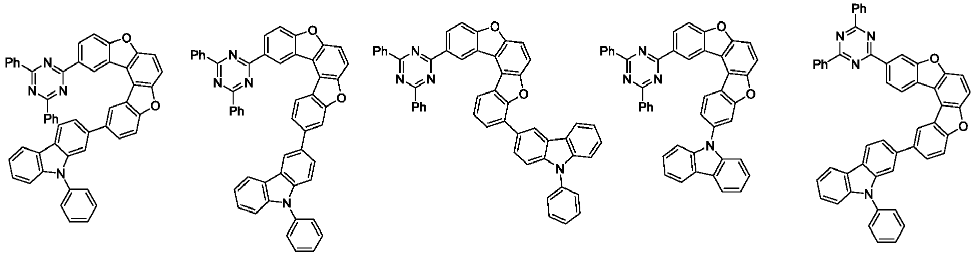

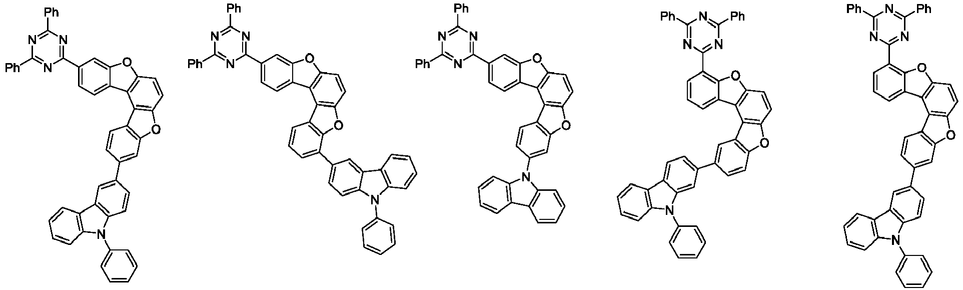

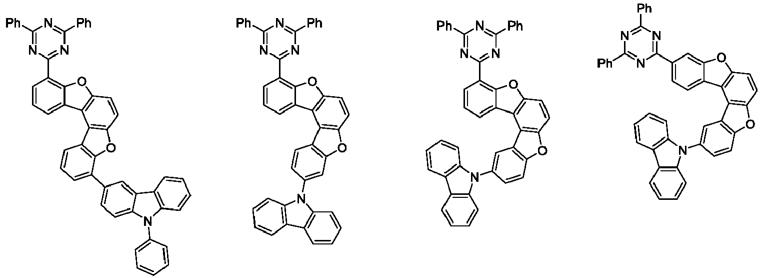

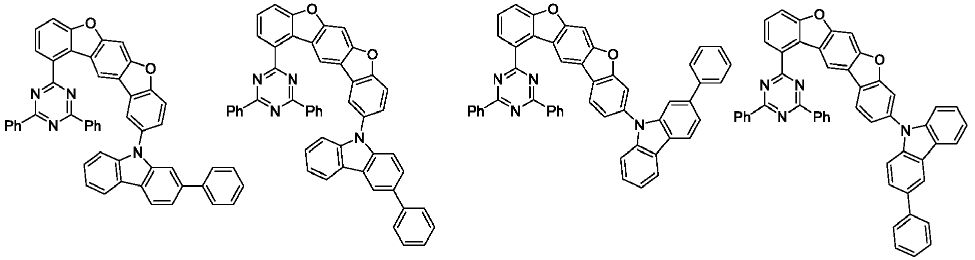

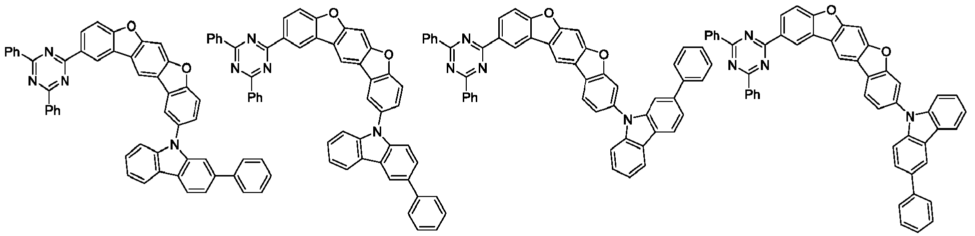

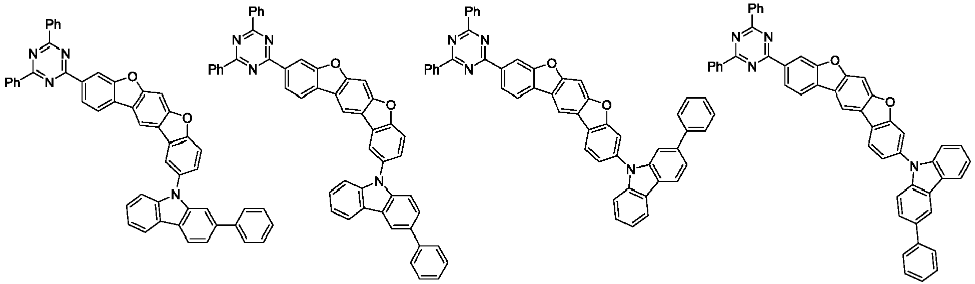

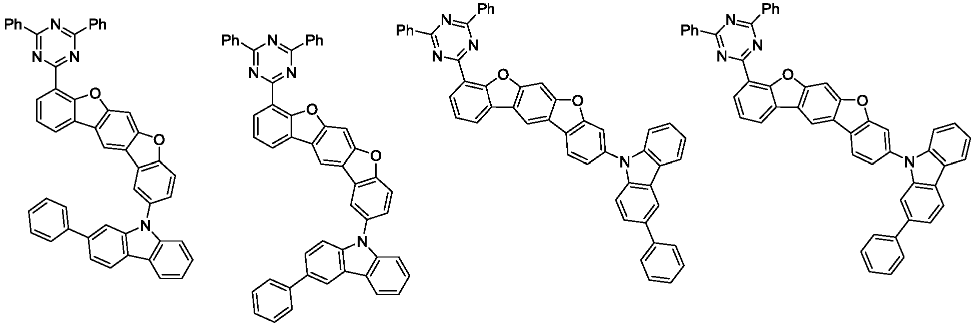

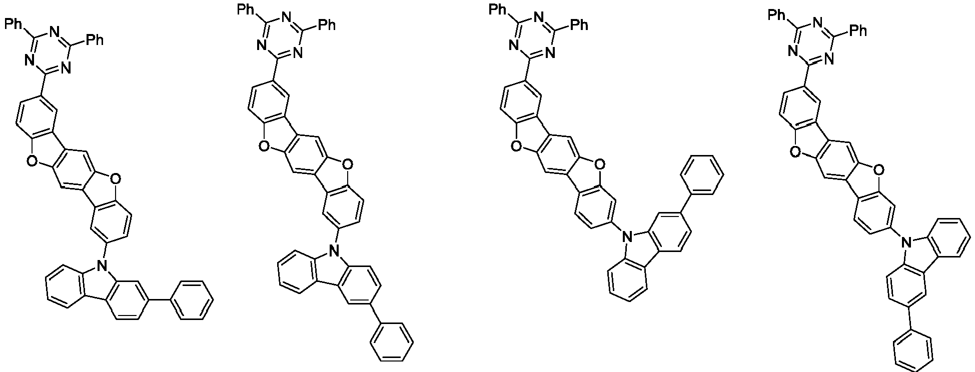

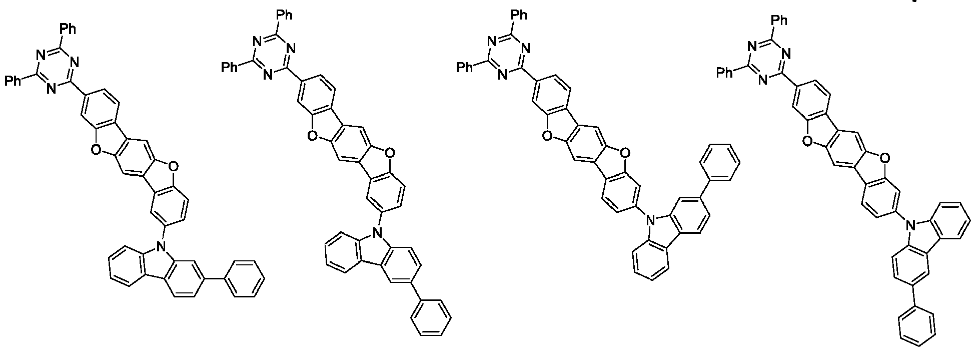

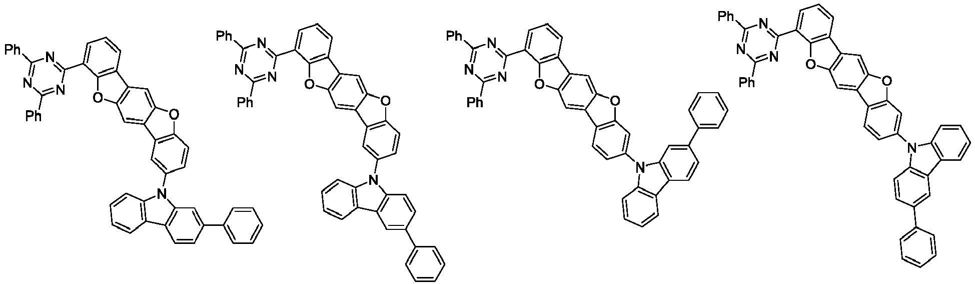

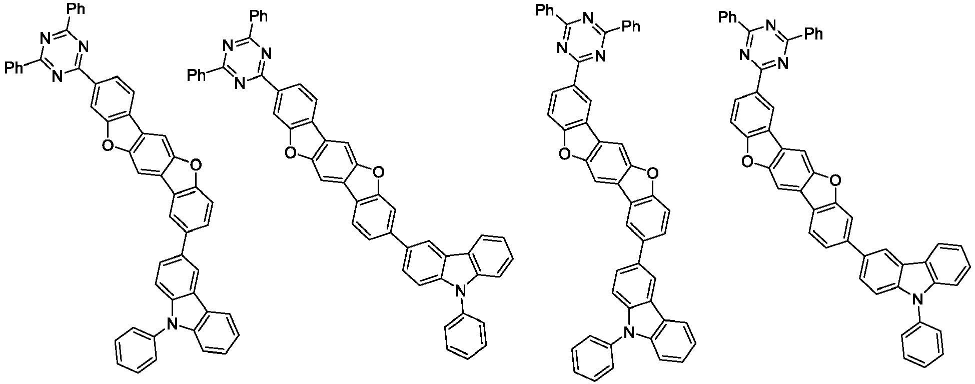

The polycyclic compounds of formulae (I) and (II) according to the present invention are particularly suitable for use in organic electronics applications, especially OLEDs, especially as a host material, a charge transporting material, charge and/or exciton blocking material, preferably having a good overall performance, especially improved efficiency and/or lifetime, preferably as host material. The structure of organic electronics applications, especially OLEDs is known in the art. Suitable structures are described below.

### Compounds of formulae (I) and (II) according to the present invention in organic electronics applications

In the following applications of the compounds of formulae (I) and (II) according to the present invention as mentioned above in organic electronic applications will be explained.

It has been found that the compounds of formulae (I) and (II) according to the present invention are particularly suitable for use in applications in which charge carrier conductivity is required, especially for use in organic electronics applications, more preferably for use in organic light-emitting diodes (OLEDs).

The compounds of formulae (I) and (II) according to the present invention being particularly suitable in OLEDs for use in a light-emitting layer, wherein the compounds of formulae (I) and (II) are preferably used as a host material, a charge transporting material, charge and/or exciton blocking material, more preferably as a host material.

In a preferred embodiment, the compounds of formulae (I) and (II) according to the present invention are present in OLEDs as a host, as a single host or as a host in combination with one or more further hosts.

The present invention therefore relates to an electronic device, preferably an organic electroluminescence device, more preferably an organic light emitting diode (OLED), comprising at least one compound of formula (I) or (II) according to the present invention.

The present invention preferably further relates to the electronic device according to the present invention, preferably an organic electroluminescence device, more preferably an organic light emitting diode (OLED), comprising a cathode, an anode, and a plurality of organic layers provided between the cathode and the anode, wherein at least one of the plurality of organic layers comprising at least one compound of formula (I) or (II) according to the present invention.

The present invention preferably further relates to the electronic device according to the present invention, preferably an organic electroluminescence device, more preferably an organic light emitting diode (OLED), comprising a cathode, an anode, and a plurality of organic layers provided between the cathode and the anode, wherein at least one of the plurality of organic layers is an emitting layer, wherein at least one organic layer, preferably the emitting layer, comprise at least one compound of formula (I) or (II) according to the present invention.

More preferably, the emitting layer comprises at least one compound of formula (I) or (II) according to the present invention as a host material.

In a further preferred embodiment, the emitting layer comprises a heavy-metal complex material.

In a further preferred embodiment, the organic layers in the electronic device according to the present invention, preferably an organic electroluminescence device, more preferably an organic light emitting diode (OLED), comprise an electron transporting layer comprising - at least one compound of formula (I) or (II) according to the present invention, said electron transporting layer placed between the cathode and the emitting layer.

Further preferably, at least one of the layers between the emitting layer and the cathode in the electronic device according to the present invention, preferably an organic electroluminescence device, more preferably an organic light emitting diode (OLED), comprises at least one compound selected from an alkali metal, an alkaline earth metal, a rare earth metal, a compound comprising an alkali metal, an alkaline earth metal, or a rare earth metal, and a complex comprising an alkali metal, an alkaline earth metal, or a rare earth metal.

The present invention further relates to an electronic equipment comprising the organic electroluminescence device according to the present invention.

The present invention also relates to an emitting layer, preferably present in an electronic device, more preferably in an electroluminescence device, particularly preferably in an organic light emitting diode (OLED), comprising at least one compound of formula (I) or (II) according to the present invention.

The present invention preferably further relates to the use of at least one compound of formula (I) or (II) according to the present invention in an electronic device, preferably in an organic electroluminescence device, particularly preferably in an organic light emitting diode (OLED), preferably in an emitting layer.

According to the present application, the terms matrix and host are used interchangeably.

Suitable structures of organic electronic devices, especially organic light-emitting diodes (OLEDs), are known to those skilled in the art and are specified below.

Examples of preferred compounds according to general formulae (I) and (II) are shown above.

Most preferably, the electronic device according to the present invention is an organic light emitting diode (OLED).

### Structure of the inventive organic EL device

The inventive organic EL device thus generally has the following structure: an anode (a) and a cathode (i) and a light-emitting layer (e) arranged between the anode (a) and the cathode (i).

The inventive organic EL device may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the organic EL device does not have all of the layers mentioned; for example, an organic EL device with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. Organic EL devices which have layers (a), (c), (e) and (i), or layers (a), (e), (f), (g) and (i), are likewise suitable. In addition, the organic EL devices may have a blocking layer for electrons/excitons (d) between the hole transport layer (c) and the light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the organic EL device among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the organic EL devices such that it is matched optimally to the organic compounds used in accordance with the invention.

In a preferred embodiment the organic EL device according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the organic EL device according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the art and are described in more detail below on basis of preferred embodiments.

In addition to the layers mentioned above, the organic EL device usually comprises a substrate adjacent to the anode.

A schematic structure of an example of the organic EL device in an aspect of the invention is for example shown in FIG. 1 wherein the organic EL device 1 comprises a substrate 2, an anode 3, a cathode 4, and an emission unit 10 disposed between the anode 3 and the cathode 4. The emission unit 10 comprises a light emitting layer 5 which comprises at least one emitting layer, preferably at least one phosphorescent emitting layer, containing a host material, preferably a phosphorescent host material, and a dopant material, preferably a phosphorescent dopant material (phosphorescent material). A hole injecting/transporting layer (an anode-side organic thin film layer) 6 may be disposed between the light emitting layer 5 and the anode 3, and an electron injecting/transporting layer (a cathode-side thin film layer) 7 may be disposed between the light emitting layer 5 and the cathode 4. An electron blocking layer may be disposed on the anode 3 side of the light emitting layer 5, and a hole blocking layer may be disposed on the cathode 4 side of the light emitting layer 5. With these blocking layers, electrons and holes are confined in the light emitting layer 5 to increase the degree of exciton generation in the light emitting layer 5.

The least one compound of formula (I) or (II) according to the present invention may be present in any layer of the organic EL device, preferably a host material, a charge transporting material, charge and/or exciton blocking material. More preferably, the least one compound of formula (I) or (II) according to the present invention are present as host material in the emitting layer of the organic EL device.

The organic EL device may have an electron transporting layer (g) between the light emitting layer (e) and the cathode (i), and the electron transporting layer may contain the polycyclic compound of formula (I) or (II) according to the present invention. Further, both the light emitting layer (e) and the electron transporting layer (g) each contain the polycyclic compound.

Alternatively, the organic EL device may have a hole transporting layer (c) between the light emitting layer and the anode, and the hole transporting layer may contain the polycyclic compound of formula (I) or (II) according to the present invention.

Further, the polycyclic compound of formula (I) or (II) according to the present invention is preferably incorporated into at least the light emitting layer (e). The polycyclic compound of formula (I) or (II) according to the present invention may additionally or alternatively be used in the electron transporting layer (g) and/or the (optionally present) electron injecting layer (h).

In particular, in addition to the electron transporting layer or the electron injecting layer, the light emitting layer preferably contains the polycyclic compound of formula (I) or (II) according to the present invention as a host material.

A multi-layer type organic EL device is obtained by laminating a plurality of layers; for example, the device is formed of an anode, a hole transporting layer (a hole injecting layer), a light emitting layer, and a cathode, of an anode, a light emitting layer, an electron transporting layer (an electron injecting layer), and a cathode, of an anode, a hole transporting layer (a hole injecting layer), a light emitting layer, an electron transporting layer (an electron injecting layer), and a cathode, or of an anode, a hole transporting layer (a hole injecting layer), a light emitting layer, a hole barrier layer, an electron transporting layer (an electron injecting layer), and a cathode.

In the organic EL device of the present invention, the light emitting layer preferably contains the polycyclic compound of formula (I) or (II) according to the present invention as a host material. In addition, it is preferred that the light emitting layer be formed of a host material and a luminescent (phosphorescent or fluorescent) material, preferably a phosphorescent material, and the host material be the polycyclic compound of formula (I) or (II) according to the present invention.

In addition, the polycyclic compound of formula (I) or (II) according to the present invention may be a host material to be used together with a luminescent (phosphorescent or fluorescent) material, preferably a phosphorescent material, or may be an electron transporting material to be used together with a luminescent (phosphorescent or fluorescent) material, preferably a phosphorescent material. The phosphorescent emitting material has a triplet energy gap of preferably 2.0 to 3.2 eV

The phosphorescent emitting material is preferably a compound containing iridium (Ir), osmium (Os), ruthenium (Ru), or platinum (Pt) because the compound has a high phosphorescent quantum yield, and can additionally improve the external quantum efficiency of the light emitting device. The material is more preferably a metal complex such as an iridium complex, an osmium complex, a ruthenium complex, or a platinum complex. Of those, the iridium complex and the platinum complex are still more preferred, and an ortho metalated iridium complex is most preferred. Specific examples of the metal complex such as an iridium complex, an osmium complex, a ruthenium complex, or a platinum complex are shown below.

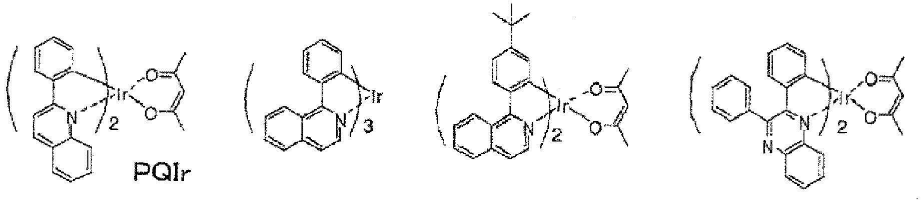

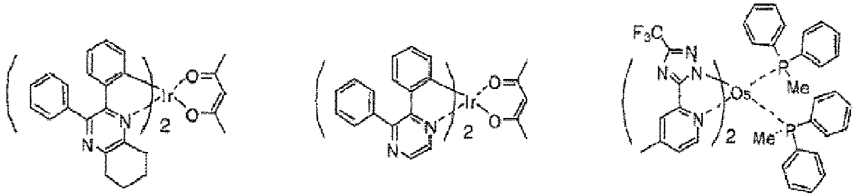

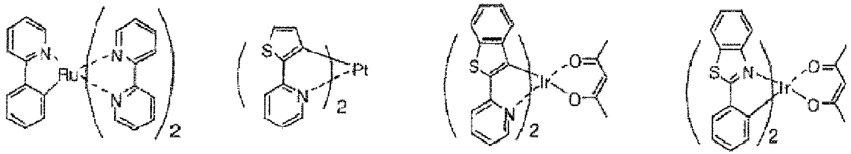

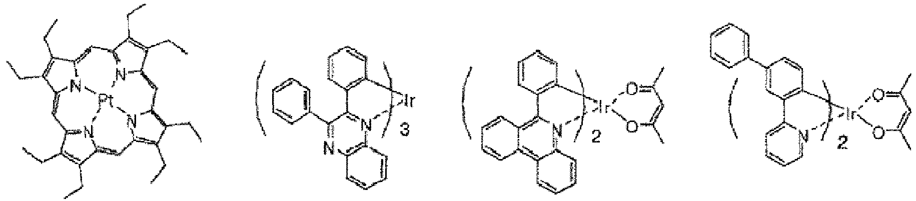

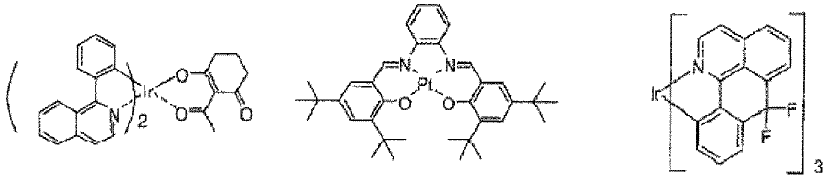

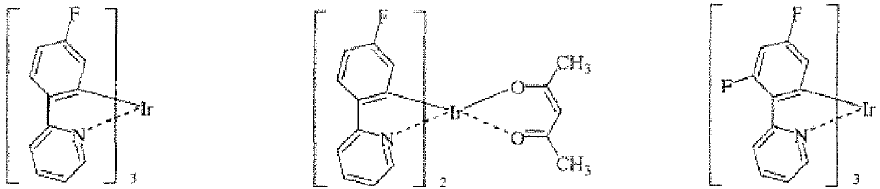

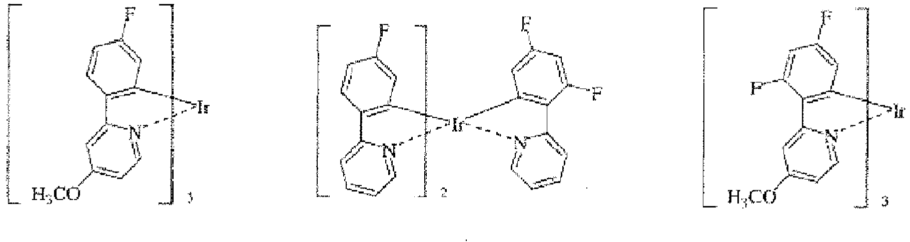

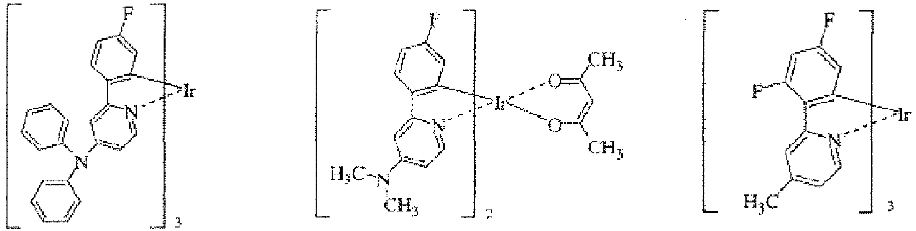

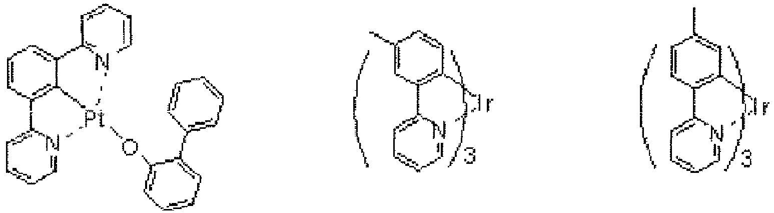

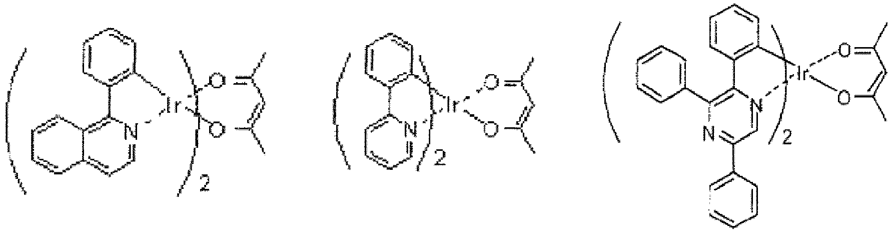

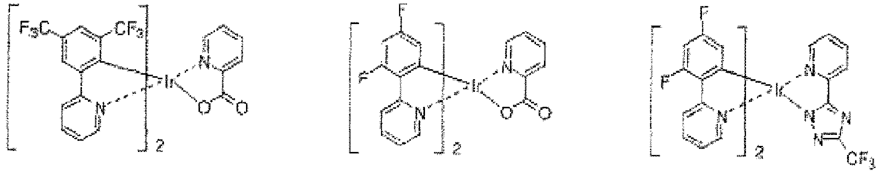

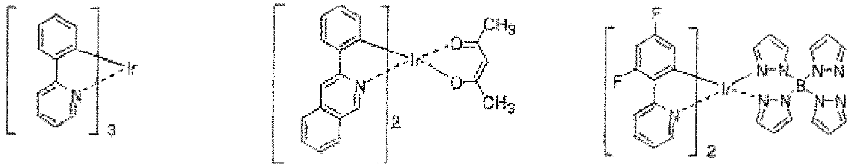

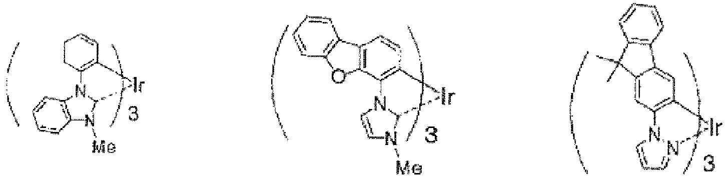

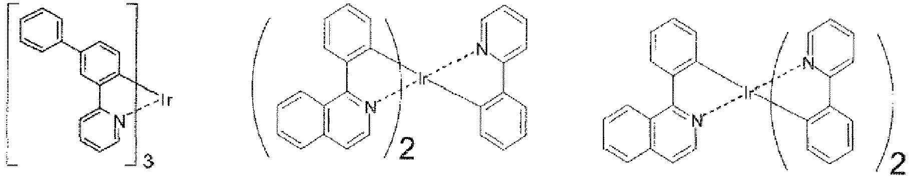

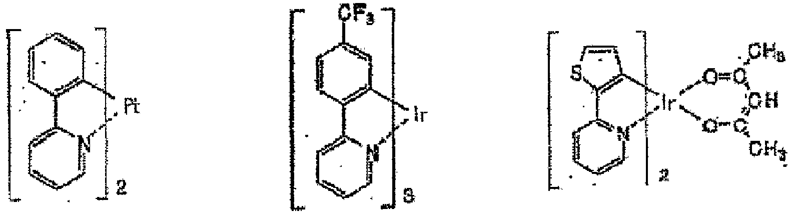

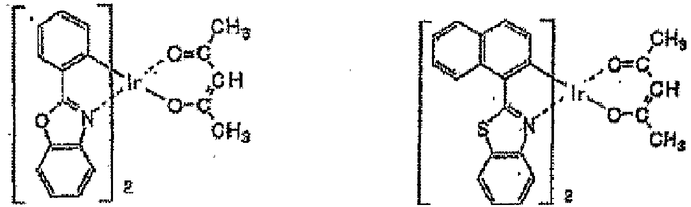

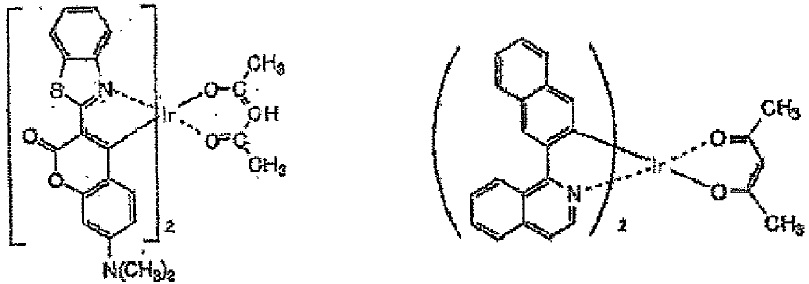

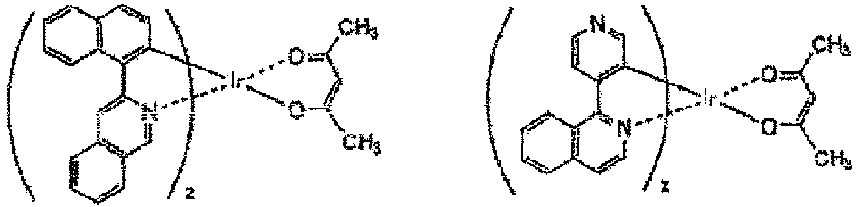

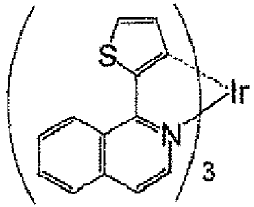

In addition, the organic EL device of the present invention is preferably such that the light emitting layer contains a host material and a phosphorescent material, and preferably contains a metal complex having a local maximum luminous wavelength of 500 to 460 nm or more.

Further, the polycyclic compound of formula (I) or (II) according to the present invention can be used together with a fluorescent dopant. The polycyclic compound of formula (I) or (II) according to the present invention can be used together with a blue, green, or red fluorescent dopant. In particular, the polycyclic compound of formula (I) or (II) according to the present invention can be more preferably used together with the blue or green fluorescent dopant. Further, the polycyclic compound of formula (I) or (II) according to the present invention can be preferably used also as an electron transporting material for a fluorescent organic EL device.

The organic EL device of the present invention preferably has a reductive dopant in an interfacial region between the cathode and an organic thin layer (for example, an electron injecting layer or a light emitting layer). Examples of the reductive dopant include at least one kind selected from an alkali metal, an alkali metal complex, an alkali metal compound, an alkaline earth metal, an alkaline earth metal complex, an alkaline earth metal compound, a rare earth metal, a rare earth metal complex, and a rare earth metal compound.

Preferred examples of the alkali metal include an alkali metal having a work function of 2.9 eV or less, such as Na having a work function of 2.36 eV, K having a work function of 2.28 eV, Rb having a work function of 2.16 eV, and Cs having a work function of 1.95 eV. Of those, K, Rb, and Cs are more preferred, Rb or Cs is still more preferred, and Cs is most preferred.

Preferred examples of the alkali earth metal include an alkali earth metal having a work function of 2.9 eV or less, such as Ca having a work function of 2.9 eV, Sr having a work function of 2.0 to 2.5 eV, and Ba having a work function of 2.52 eV.

Preferred examples of the rare earth metal include a rare earth metal having a work function of 2.9 eV or less, such as Sc, Y, Ce, Tb, and Yb.

Of those metals, a preferred metal has a particularly high reductive ability, so improvement of light emission intensity and long life of organic EL device can be attained by adding a relatively small amount of the metal to an electron injecting region.

Examples of the alkali metal compound include an alkali oxide such as Li₂O, Cs₂O, or K₂O, and an alkali halide such as LiF, NaF, CsF, or KF. Of those, LiF, Li₂O, and NaF are preferred. Examples of the alkali earth metal compound include BaO, SrO, CaO, admixtures thereof such as BaₘSr₁₋ₘO (0<m<1) and BaₘCa₁₋ₘO (0<m<1). Of those, BaO, SrO, and CaO are preferred. Examples of the rare earth metal compound include YbF₃, ScF₃, SicO₃, Y₂O₃, Ce₂O₃, GdF₃, and TbF₃. Of those, YbF₃, ScF₃, and TbF₃ are preferred.

The alkali metal complex, alkali earth metal complex, and rare earth metal complex are not particularly limited as long as they each include as a metal ion at least one of alkali metal ions, alkali earth metal ions, and rare earth metal ions. Meanwhile, preferred examples of a ligand include, but not limited to, quinolinol, benzoquinolinol, acridinol, phenanthridinol, hydroxy-phenyloxazole, hydroxyphenylthiazole, hydroxydiaryloxadiazole, hydroxydiarylthiadiazole, hy-droxyphenylpyridine, hydroxyphenylbenzoimidazole, hydroxybenzotriazole, hydroxyfluborane, bipyridyl, phenanthroline, phthalocyanine, porphyrin, cyclopentadiene, β-diketones, azomethines, and derivatives thereof.

For the addition form of the reductive dopant, it is preferred that the reductive dopant be formed in a shape of a layer or an island in the interfacial region. A preferred example of the forming method includes a method in which an organic substance which is a light emitting material or an electron injecting material for forming the interfacial region is deposited at the same time as the reductive dopant is deposited by a resistant heating deposition method, thereby dispersing the reductive dopant in the organic substance. The disperse concentration by molar ratio of the organic substance to the reductive dopant is 100:1 to 1:100, and is preferably 5:1 to 1:5.

In a case where the reductive dopant is formed into the shape of a layer, the light emitting material or electron injecting material which serves as an organic layer in the interface is formed into the shape of a layer. After that, the reductive dopant is solely deposited by the resistant heating deposition method to form a layer preferably having a thickness of 0.1 to 15 nm.

In a case where the reductive dopant is formed into the shape of an island, the light emitting material or electron injecting material which serves as an organic layer in the interface is formed into the shape of an island. After that, the reductive dopant is solely deposited by the resistant heating deposition method to form an island preferably having a thickness of 0.05 to 1 nm.

When the organic EL device of the present invention has an electron injecting layer between the light emitting layer and the cathode, an electron transporting material to be used in the electron injecting layer is preferably an aromatic heterocyclic compound containing one or more hetero atoms in any one of its molecules, or particularly preferably a nitrogen-containing ring derivative.

The nitrogen-containing ring derivative is preferably, for example, a nitrogen-containing ring metal chelate complex represented by the following formula (A).

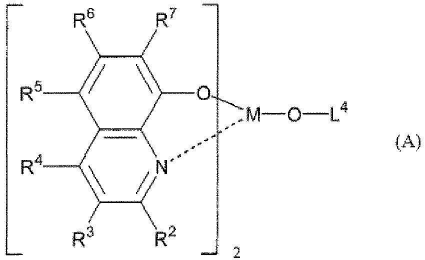

R² to R⁷ each independently represent a hydrogen atom, a halogen atom, an amino group, a hydrocarbon group each having 1 to 40 carbon atoms, an alkoxy group, an aryloxy group, an alkoxycarbonyl group, or a heterocyclic group, each of which may be substituted.

M represents aluminum (Al), gallium (Ga), or indium (In). Indium is preferred.
L⁴ in the formula (A) is a group represented by the following formula (A') or (A").

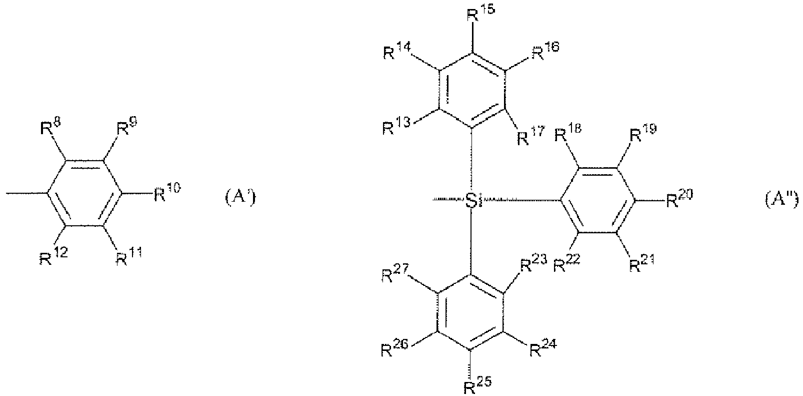

(In the formula (A'), R⁸ to R¹² each independently represent a hydrogen atom, or a substituted or unsubstituted hydrocarbon group having 1 to 40 carbon atoms, and adjacent groups may form a cyclic structure. In addition, in the formula (A"), R¹³ to R²⁷ each independently represent a hydrogen atom, or a substituted or unsubstituted hydrocarbon group having 1 to 40 carbon atoms, and adjacent groups may form a cyclic structure.)

A nitrogen-containing heterocyclic derivative is a nitrogen-containing heterocyclic derivative formed of an organic compound having any one of the following formulae, and a nitrogen-containing compound which is not a metal complex is also an example of the derivative. Examples of the derivative include a five- or six-membered ring containing a skeleton represented by the following formula (a) and a derivative of a structure represented by the following formula (b).

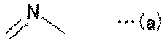

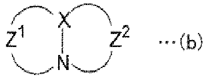

(In the formula (b), X represents a carbon atom or a nitrogen atom, and Z¹ and Z² each independently represent an atomic group capable of forming a nitrogen-containing heterocycle.)

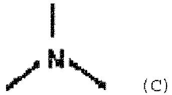

An organic compound having a nitrogen-containing aromatic polycycle formed of a five - or six-membered ring is preferred. In the case of such nitrogen-containing aromatic polycycle having a plurality of nitrogen atoms, a nitrogen-containing aromatic polycyclic organic compound having a skeleton obtained by combining the above-mentioned formulae (a) and (b) or the above-mentioned formulae (a) and (c) is more preferred.

The nitrogen-containing group of the nitrogen-containing organic compound is selected from, for example, nitrogen-containing heterocyclic groups represented by the following formulae.

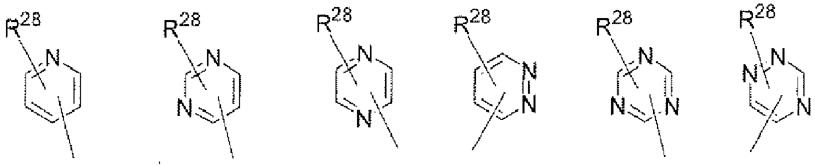

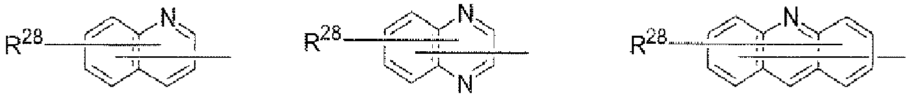

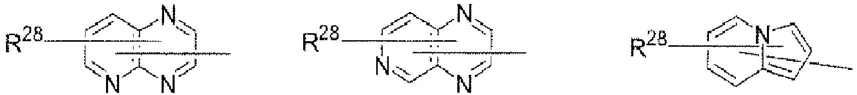

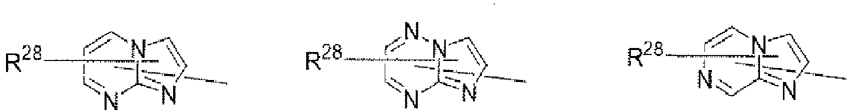

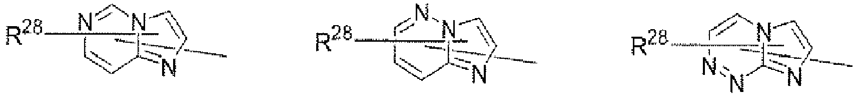

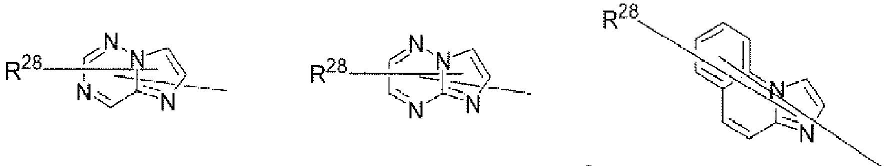

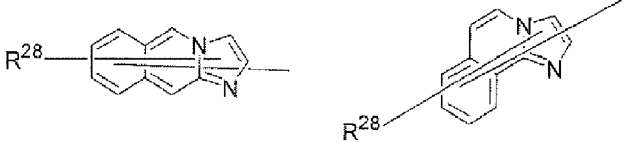

(In each of the formulae, R²⁸ represents an aryl group having 6 to 40 carbon atoms, a heteroaryl group having 3 to 40 carbon atoms, an alkyl group having 1 to 20 carbon atoms, or an alkoxy group having 1 to 20 carbon atoms, when the number of R²⁸ is two or more, a plurality of R²⁸s may be identical to or different from each other.)

Further, a preferred specific compound is, for example, a nitrogen-containing heterocyclic derivative represented by the following formula.

HAr^{a}-L⁶-Ar^{b}-Ar^{c}

(In the formula, HAr^{a} represents a nitrogen-containing heterocycle which has 3 to 40 carbon atoms and which may have a substituent, L⁶ represents a single bond, an arylene group which has 6 to 40 carbon atoms and which may have a substituent, or a heteroarylene group which has 3 to 40 carbon atoms and which may have a substituent, Ar^{b} represents a divalent aromatic hydrocarbon group which has 6 to 40 carbon atoms and which may have a substituent, and Ar^{c} represents an aryl group which has 6 to 40 carbon atoms and which may have a substituent, or a heteroaryl group which has 3 to 40 carbon atoms and which may have a substituent.)

HAr^{a} is selected from, for example, the following groups.

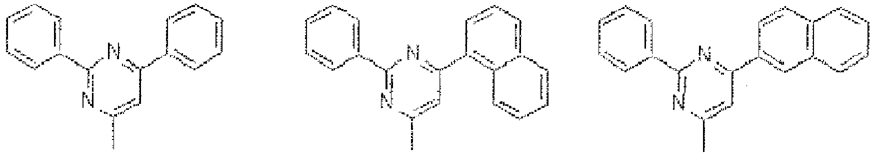

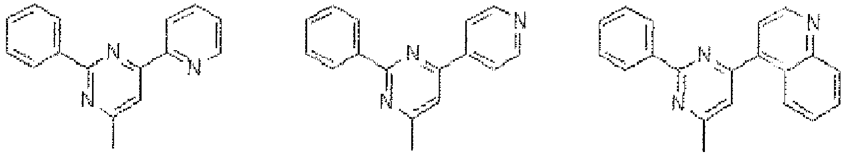

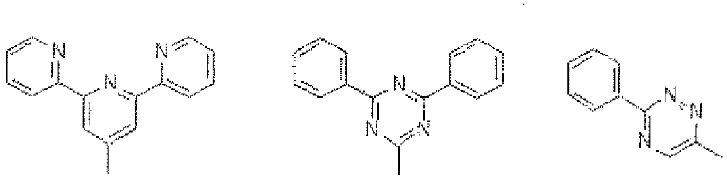

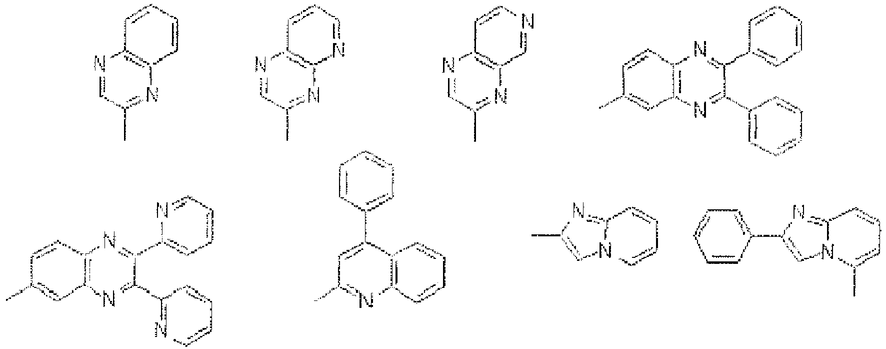

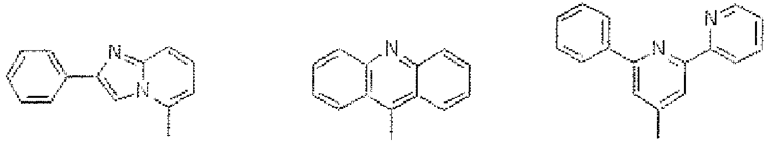

L⁶ is selected from, for example, the following groups.

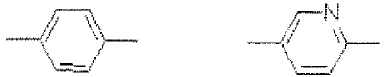

Ar^{c} is selected from, for example, the following groups.

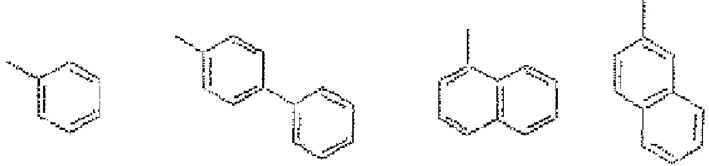

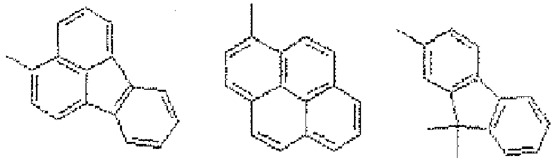

Ar^{b} is selected from, for example, the following arylanthranil groups.

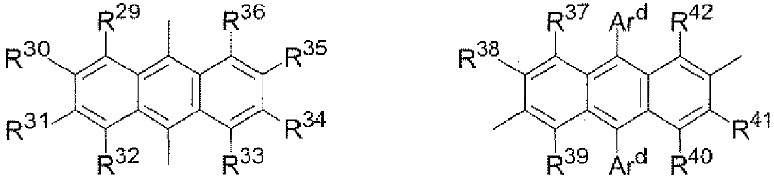

(In the formulae, R²⁹ to R⁴² each independently represent a hydrogen atom, a halogen atom, an alkyl group having 1 to 20 carbon atoms, an alkoxy group having 1 to 20 carbon atoms, an aryloxy group having 6 to 40 carbon atoms, an aryl group which has 6 to 40 carbon atoms and which may have a substituent, or a heteroaryl group having 3 to 40 carbon atoms, and Ar^{d} represents an aryl group which has 6 to 40 carbon atoms and which may have a substituent, or a heteroaryl group having 3 to 40 carbon atoms.)

In addition, a nitrogen-containing heterocyclic derivative in which R²⁹ to R³⁶ in Ar^{b} represented by the above-mentioned formula each represent a hydrogen atom is preferred.

In addition to the foregoing, the following compound (see JP 09-3448 A) is also suitably used.

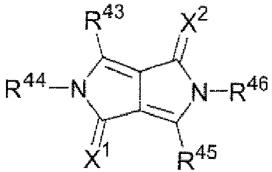

(In the formula, R⁴³ to R⁴⁶ each independently represent a hydrogen atom, a substituted or unsubstituted aliphatic group, a substituted or unsubstituted alicyclic group, a substituted or unsubstituted carbocyclic aromatic ring group, or a substituted or unsubstituted heterocyclic group, and X¹ and X² each independently represent an oxygen atom, a sulfur atom, or a dicyanomethylene group.)

In addition, the following compound (see JP 2000-173774 A) is also suitably used.

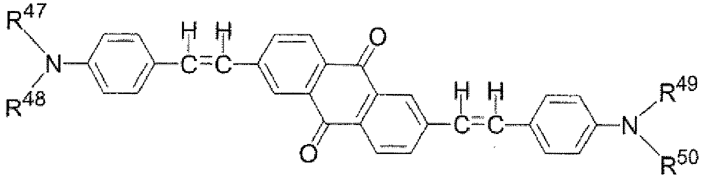

(In the formula, R⁴⁷, R⁴⁸, R⁴⁹, and R⁵⁰ represent groups identical to or different from one another, and each represent an aryl group represented by the following formula.)

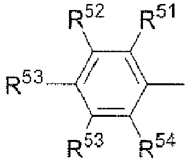

(In the formula, R⁵¹, R⁵², R⁵³, R⁵⁴, and R⁵⁵ represent groups identical to or different from one another, and each represent a hydrogen atom, or at least one of them may represent a saturated or unsaturated alkoxyl, alkyl, amino, or alkylamino group.)

Further, a polymer compound containing the nitrogen-containing heterocyclic group or nitrogen-containing heterocyclic derivative is also permitted.

In addition, the electron transporting layer preferably contains at least one of the nitrogen-containing heterocyclic derivatives represented by the following formulae (201) to (203).

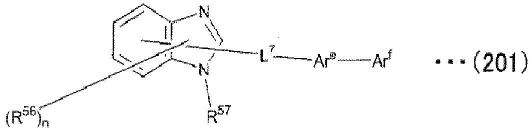

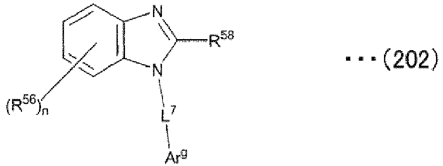

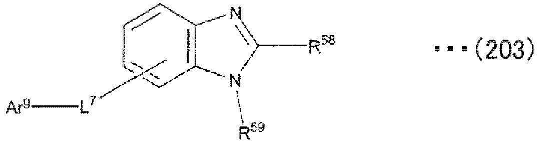

In the formulae (201) to (203), R⁵⁶ represents a hydrogen atom, an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group which has 1 to 20 carbon atoms and which may have a substituent, n represents an integer of 0 to 4, R⁵⁷ represents an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group having 1 to 20 carbon atoms, R⁵⁸ and R⁵⁹ each independently represent a hydrogen atom, an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group which has 1 to 20 carbon atoms and which may have a substituent, L⁷ represents a single bond, an arylene group which has 6 to 60 carbon atoms and which may have a substituent, a pyridinylene group which may have a substituent, a quinolinylene group which may have a substituent, or a fluorenylene group which may have a substituent, Ar^{e} represents an arylene group which has 6 to 60 carbon atoms and which may have a substituent, a pyridinylene group which may have a substituent, or a quinolinylene group which may have a substituent, and Ar^{f} represents a hydrogen atom, an aryl group which has 6 to 60 carbon atoms and which may have a substituent, a pyridyl group which may have a substituent, a quinolyl group which may have a substituent, an alkyl group which has 1 to 20 carbon atoms and which may have a substituent, or an alkoxy group which has 1 to 20 carbon atoms and which may have a substituent.
Ar^{g} represents an aryl group which has 6 to 60 carbon atoms and which may have a substituent, a pyridyl group which may have a substituent, a quinolyl group which may have a substituent, an alkyl group which has to 20 carbon atoms and which may have a substituent, an alkoxy group which has 1 to 20 carbon atoms and which may have a substituent, or a group represented by -Ar^{e}- Ar^{f} (Ar^{e} and Ar^{f} each have the same meaning as that described above).

In addition, as the nitrogen-containing ring derivative, nitrogen-containing five-membered ring derivative are preferably exemplified. Examples of the nitrogen-containing five-membered ring include an imidazole ring, a triazole ring, a tetrazole ring, an oxadiazole ring, a thiadiazole ring, an oxatriazole ring, and a thiatriazole ring. Examples of the nitrogen-containing five-membered ring derivative include a benzoimidazole ring, a benzotriazole ring, a pyridinoimidazole ring, a pyrimidinoimidazole ring, and a pyridazinoimidazole ring. Particularly preferred is the compound represented by the following formula (B).

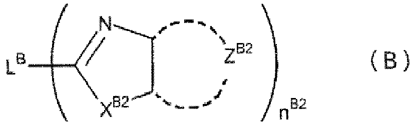

In the formula (B), L^{B} represents a divalent or more bonding group. Examples thereof include a carbon atom, a silicon atom, a nitrogen atom, a boron atom, an oxygen atom, a sulfur atom, metal atoms (for example, a barium atom, a beryllium atom), aromatic hydrocarbon rings, and aromatic heterocycles.

Of the nitrogen-containing five-membered ring derivatives each represented by the formula (B), a derivative represented by the following formula (B') is more preferred.

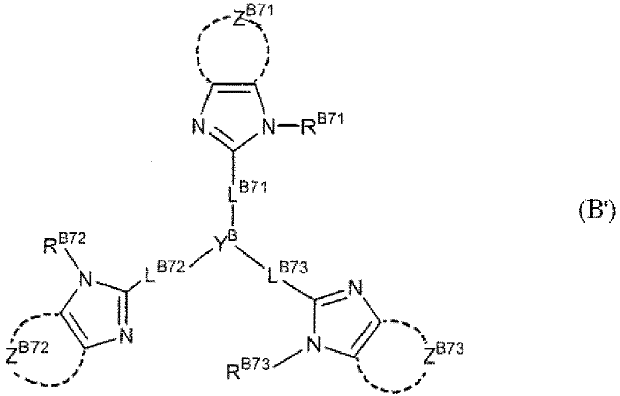

In the formula (B'), R^{B71}, R^{B72}, and R^{B73} each have the same meaning as that of R^{B2} in the formula (B).
Z^{B71}, Z^{B72}, and Z^{B73} each have the same meaning as that of Z^{B2} in the formula (B).
L^{B71}, L^{B72} and L^{B73} each represent a linking group, and examples of the linking group include examples obtained by making the examples of L^{B} in the formula (B) divalent. The linking group is preferably a single bond, a divalent aromatic hydrocarbon ring group, a divalent aromatic heterocyclic group, or a linking group formed of a combination of two or more of them, or is more preferably a single blond. L^{B71}, L^{B72}, and L^{B73} may each have a substituent. Examples of the substituent include the same examples as those described for the substituent of the group represented by L^{B} in the formula (B).
Y^{B} represents a nitrogen atom, a 1,3,5-benzenetriyl group, or a 2,4,6-triazinetriyl group.

A compound of which each of the electron injecting layer and the electron transporting layer is formed is, for example, a compound having a structure obtained by combining an electron-deficient, nitrogen-containing five-membered ring skeleton or electron-deficient, nitrogen-containing six-membered ring skeleton and a substituted or unsubstituted indole skeleton, substituted or unsubstituted carbazole skeleton, or substituted or unsubstituted azacarbazole skeleton as well as the polycyclic compound of the present invention. In addition, a suitable electron-deficient, nitrogen-containing five-membered ring skeleton or electron-deficient, nitrogen-containing six-membered ring skeleton is a molecular skeleton such as a pyridine, pyrimidine, pyrazine, triazine, triazole, oxadiazole, pyrazole, imidazole, quinoxaline, or pyrrole skeleton, or benzimidazole or imidazopyridine obtained when two or more of them fuse with each other. Of those combinations, a preferred combination is, for example, a combination of a pyridine, pyrimidine, pyrazine, or triazine skeleton and a carbazole, indole, azacarbazole, or quinoxaline skeleton. The skeleton may be substituted or unsubstituted.

Each of the electron injecting layer and the electron transporting layer may be of a monolayer structure formed of one or two or more kinds of the materials, or may be of a multi-layered structure formed of the plurality of layers identical to or different from each other in composition. Materials for those layers each preferably have an n-electron-deficient, nitrogen-containing heterocyclic group.

In addition, an insulator or semiconductor serving as an inorganic compound as well as the nitrogen-containing ring derivative is preferably used as a component of the electron injecting layer. When the electron injecting layer is formed of an insulator or semiconductor, current leakage can be effectively prevented, and the electron injecting property of the layer can be improved.
As the insulator, at least one metal compound selected from the group consisting of alkali metal chalcogenides, alkaline earth metal chalcogenides, alkali metal halides, and alkaline earth metal halides is preferably used. It is preferred that the electron injecting layer be formed of the alkali metal chalcogenide or the like since the electron injecting property can be further improved. To be specific, preferred examples of the alkali metal chalcogenide include Li₂O, K₂O, Na₂S, Na₂Se, and Na₂O, and preferred examples of the alkaline earth metal chalcogenide include CaO, BaO, SrO, BeO, BaS, and CaSe. In addition, preferred examples of the alkali metal halide include LiF, NaF, KF, LiCl, KCl, and NaCl. Further, preferred examples of the alkaline earth metal halide include fluorides such as CaF₂, BaF₂, SrF₂, MgF₂, and BeF₂ and halides other than the fluorides.
In addition, examples of the semiconductor include oxides, nitrides, and oxide nitrides containing at least one element selected from the group consisting of Ba, Ca, Sr, Yb, Al, Ga, In, Li, Na, Cd, Mg, Si, Ta, Sb, and Zn. One kind of them may be used alone, or two or more kinds of them may be used in combination. In addition, it is preferred that the inorganic compound composing the electron injecting layer form a crystallite or amorphous insulating thin film. When the electron injecting layer is formed of the insulating thin film, a more uniform thin film can be formed, and defects of pixels such as dark spots can be decreased. It should be noted that examples of the inorganic compound include alkali metal chalcogenides, alkaline earth metal chalcogenides, alkali metal halides, and alkaline earth metal halides.
In addition, the reducing dopant can be preferably incorporated into the electron injecting layer in the present invention.
It should be noted that the thickness of each of the electron injecting layer and the electron transporting layer, which is not particularly limited, is preferably 1 to 100 nm.

An aromatic amine compound such as an aromatic amine derivative represented by the formula (I) is suitably used in the hole injecting layer or hole transporting layer (a hole injecting/transporting layer is also included in this category).

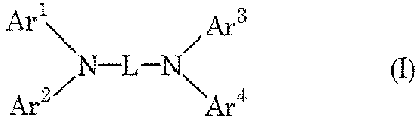

(In the formula (I) Ar¹ to Ar⁴ each represent a substituted or unsubstituted aryl group having a ring formed of 6 to 50 carbon atoms, or a substituted or unsubstituted heteroaryl group having a ring formed of 5 to 50 atoms.)

L represents a linking group, and specifically, a substituted or unsubstituted arylene group having a ring formed of 6 to 50 carbon atoms, a substituted or unsubstituted heteroarylene group having a ring formed of 5 to 50 atoms, or a divalent group in which two or more arylene groups or heteroarylene groups are bonded by a single bond, an ether bond, a thioether bond, with an alkylene group having 1 to 20 carbon atoms, an alkenylene group having 2 to 20 carbon atoms, and an amino group.

In addition, an aromatic amine represented by the following formula (II) is also suitably used in the formation of the hole injecting layer or hole transporting layer.

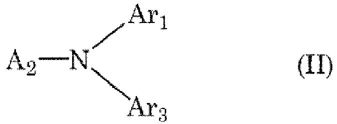

(In the formula (II), the definition of Ar¹ to Ar³ is the same as that of Ar¹ to Ar⁴ in the formula (I).)

The compound of the present invention can be used in each of the hole injecting layer, the hole transporting layer, the electron injecting layer, and the electron transporting layer, because the compound can transport both a hole and an electron.

In the present invention, the anode in the organic EL device has the function of injecting holes into the hole transporting layer or the light emitting layer. It is effective that the anode has a work function of 4.5 eV or more. Specific examples of the material for the anode used in the present invention include indium tin oxide alloys (ITO), tin oxide (NESA), gold, silver, platinum, and copper. In addition, as the cathode, a material having a small work function is preferred in view to inject an electron into an electron-injecting layer or a light-emitting layer. Examples of the cathode material are not particularly limited, and specifically, indium, aluminum, magnesium, a magnesium-indium alloy, a magnesium-aluminum alloy, an aluminum-lithium alloy, an aluminum-scandium-lithium alloy, and a magnesium-silver alloy may be used.

The method of forming the layers in the organic EL device of the present invention is not particularly limited. A conventionally known process such as the vacuum vapor deposition process or the spin coating process can be used. The organic thin film layer which is used in the organic EL device of the present invention and includes the compound represented by formula (I) or (II) according to the present invention can be formed in accordance with a known process such as the vacuum vapor deposition process or the molecular beam epitaxy process (MBE process) or, using a solution prepared by dissolving the compounds into a solvent, in accordance with a coating process such as the dipping process, the spin coating process, the casting process, the bar coating process, or the roll coating process.

The thickness of each layer in the organic thin film layer in the organic EL device of the present invention is not particularly limited. In general, an excessively thin layer tends to have defects such as pin holes, whereas an excessively thick layer requires a high applied voltage to decrease the efficiency. Therefore, a thickness in the range of several nanometers to 1 µm is typically preferred.

### Examples

Next, the present invention is described in detail by way of examples, but the present invention is not limited to the following examples. Note that, in the synthetic examples below, DMF refers to dimethylformamide, THF refers to tetrahydrofuran, DME refers to dimethoxyethane, NBS refers to N-bromosuccinimide, Ph refers to a phenyl group, EtOAc refers to ethyl acetate, MTBE refers to methyl tertiary butyl ether, DCM refers to dichloromethane and NMP refers to N-methyl pyrrolidone, Ar refers to Argon. DDQ refers to 2,3-dichloro-5,6-dicyano-1,4-benzoquinone. ¹H NMR refers to proton NMR.

### Example 1

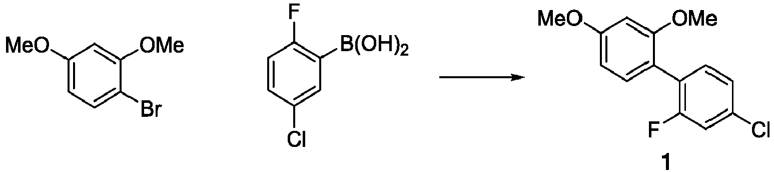

(2,4-dimethoxyphenyl)boronic acid (12 g, 65.9 mmol) was combined with 2-bromo-4-chloro-1-fluorobenzene (13.81 g, 65.9 mmol) in the presence of potassium carbonate (22.78 g, 165 mmol) in a solution of dioxane (64 ml), water (48 ml) and toluene (80 ml) and degassed under a stream of Ar for 30 minutes. Tetrakis(triphenylphosphine)palladium(0) (0.76 g, 0.66 mmol) was then added and again degassed with Ar for 30 minutes. The reaction was heated under reflux for 16 hours and then allowed to cool to room temperature. The reaction mixture was partitioned and the aqueous phase was separated. The organic phase was then washed with water, dried over Na₂SO₄ and the solvent was evaporated. The crude product was purified by chromatography on silica using 20 % toluene in heptane as eluant. Compound **1** (10.7 g, 61 % yield) was thus obtained as a colourless oil. ¹H NMR (300 MHz, DMSO-*d*₆) δ 7.48 - 7.09 (m, 4H), 6.73 - 6.57 (m, 2H), 3.82 (s, 3H), 3.76 (s, 3H).

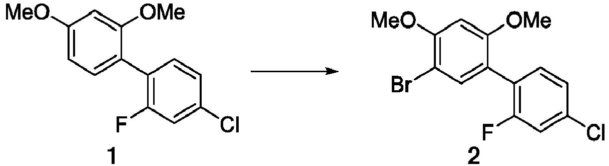

Compound **1** (10.7 g, 40.1 mmol) was added to acetic acid (30 mL) at room temperature and a solution of NBS (7.1 g, 40 mmol) in acetic acid (7 mL) was added. The resulting solution was allowed to stir at room temperature for 1 hour. The product crystallized from the reaction solution and was isolated by filtration. The crude product was washed with methanol and then dried. Compound **2** (11.25 g, 81 % yield) was isolated as a white solid. ¹H NMR (300 MHz, DMSO-*d*₆) δ 7.51 - 7.37 (m, 3H), 7.38 - 7.24 (m, 1H), 6.88 (s, 1H), 3.96 (s, 3H), 3.82 (s, 3H).

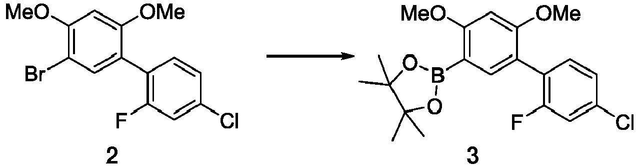

Compound **2** (11.25 g, 32.6 mmol) was combined with bis(pinacolato)diboron (9.9 g, 39 mmol) and potassium acetate (12.78 g, 130 mmol) in DMF (60 ml) and degassed for 30 minutes with Ar. (dppf)₂PdCl₂ (1 g, 1.3 mmol) was then added and the reaction mixture further degassed with Ar. The reaction was then warmed at an oil bath temperature of 90 °C for 16 hours. The reaction mixture was then filtered and diluted with MTBE and washed with water, dried over Na₂SO₄ and the solvent was evaporated under reduced pressure. The crude product was stirred in pentane and filtered twice. Compound **3** (6.2 g, 48.5 % yield) was isolated and used without further purification. ¹H NMR (300 MHz, DMSO-*d*₆) δ 7.41 - 7.22 (m, 4H), 6.71 (s, 1H), 3.84 (d, J= 4.5 Hz, 6H), 1.26 (s, 12H).

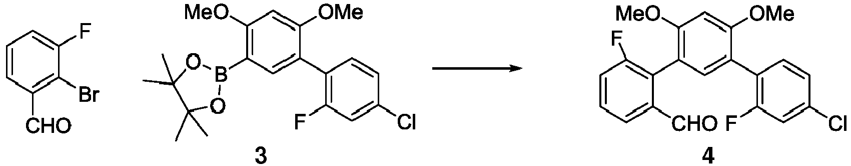

Compound **3** (6.2 g, 15.9 mmol) was combined with 2-bromo-3-fluorobenzaldehyde (3.2 g, 15.8 mmol) and potassium carbonate (5.4 g, 39.5 mmol) in a solution of toluene (50 ml), dioxane (30 ml) and water (20 ml) and degassed for 30 minutes under Ar. Tetrakis(triphenylphosphine)-palladium (0.73 g, 0.63 mmol) was then added and the reaction mixture further degassed under Ar for 30 minutes. The reaction mixture was then heated at an internal temperature of 82 °C for 16 hours. The aqueous phase was partitioned and separated. The organic phase was then washed a further 3 times with water, dried over Na₂SO₄ and the solvent was evaporated under reduced pressure. The crude product was purified by chromatography on silica using a gradient eluant system of 20 % to 80% toluene in heptane. Compound **4** (3.9 g, 63.0 % yield) was thus obtained. 1H NMR (300 MHz, DMSO-*d*6) δ 9.72 (d, *J* = 0.6 Hz, 1H), 7.79 - 7.68 (m, 1H), 7.73 - 7.51 (m, 2H), 7.52 - 7.37 (m, 2H), 7.43 - 7.19 (m, 2H), 6.94 (s, 1H), 3.87 (d, *J* = 21.3 Hz, 6H).

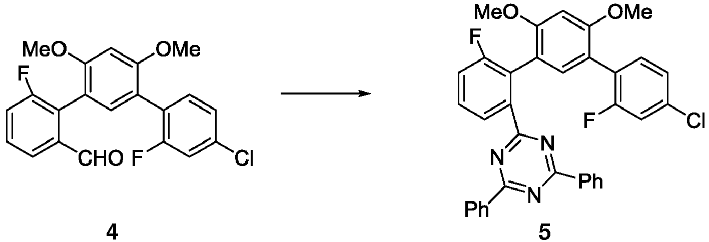

Compound **4** (3.6g, 9.2 mmol) was combined with benzimidamide hydrochloride (3.2 g, 20.4 mmol) and cesium carbonate (10.5 g, 32.4 mmol) in xylene (40 ml) and heated at an internal temperature of 120 °C for 16 hours. The hot reaction mixture was then filtered. The solution was allowed to cool to room temperature and DDQ (2.4 g, 6.1 mmol) was added. The reaction was allowed to stir at room temperature for 30 minutes. The reaction mixture was filtered and the solvent evaporated under reduced pressure. The product was purified by chromatography on silcia with 20 % toluene in heptane as eluant. 4.6 g (84 % yield) of Compound **5** was thus obtained. Product confirmed by mass spectrometry 593 (M+1).

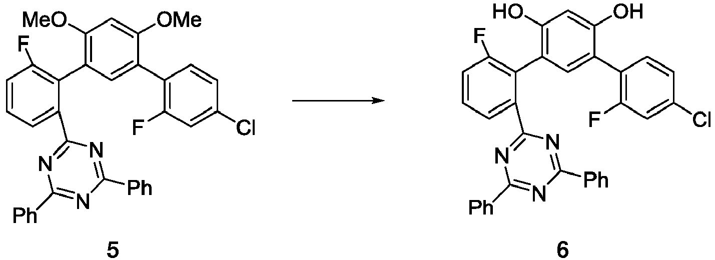

Compound **5** (4.6 g, 7.7 mmol) was dissolved in CH₂Cl₂ (20 mL) and stirred at 0 °C. BBr₃ (18.8 mL, 1M in DCM) was then added and the reaction mixture was allowed at 0°C for 1 hour. A further aliquot of BBr₃ (1.8 mL, 1M in DCM) was added and the reaction was allowed to warm to room temperature. The reaction mixture was diluted with 500 mL of 5 % NaHCO₃ and extracted with EtOAc. The organic phase was washed a further 3 times with water, dried over MgSO₄ and solvent evaporated. The crude product was purified by chromatography on silica using 20 % EtOAc in heptane as eluant. Compound **6** (3.5 g, 6.2 mmol, 80 % yield) was thus obtained and used without further purification. 1H NMR (300 MHz, DMSO-*d*6) δ 9.58 (s, 1H), 9.37 (s, 1H), 8.56 - 8.31 (m, 4H), 8.13 (m, 1H), 7.81 - 7.47 (m, 8H), 7.44 - 7.21 (m, 3H), 7.12 (s, 1H), 6.40 (s, 1H).

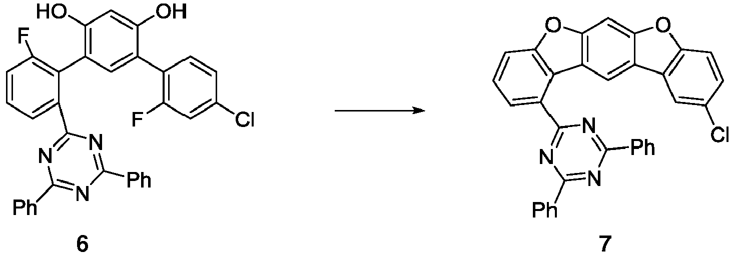

Compound **6** (3.5 g, 6.2 mmol) was combined with potassium phosphate (3.9 g, 18.6 mmol) in NMP (25 ml) and heated at an oil bath temperature of 150 °C. After 1 hour the reaction was complete and the reaction mixture was cooled to 50 °C and the product filtered. The product was then washed with water and finally dried to give the desired Compound **7** (2.9 g, 89 % yield) as a white solid. The product was used without further purification. Mass spectrometry confirmed the product 635 (M+1).

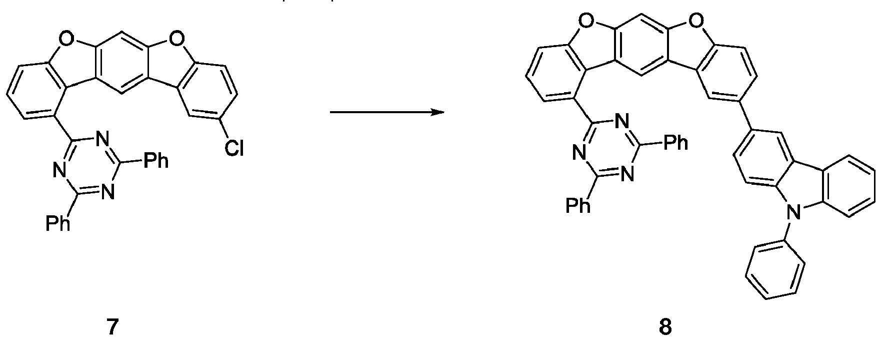

(9-phenyl-9H-carbazol-3-yl)boronic acid (1.4 g, 4.8 mmol) was combined with compound **7** (2.0 g, 3.8 mmol) and Cs₂CO₃ (2.5 g, 7.6 mmol) in a solution of ethanol (11.7 ml), water (8.8 ml) and toluene (17.6 ml) and degassed for 30 minutes under Ar. PdOAc₂ (0.021 g, 0.095 mmol) and XPhos (0.18 g, 0.38 mmol) were then added and the reaction mixture was degassed for a further 30 minutes with Ar. The reaction was heated under reflux for 3 hours and then allowed to cool to 50 °C. The reaction mixture was diluted with CHCl₃ (100 ml) and washed twice with water. The solution was then filtered over a pad of silica and the solvent removed under a reduced pressure until there was 25 mL remaining. The white suspension that formed was then filtered and washed with acetone. The product Compound **8** was thus isolated (2.4 g, 87 % yield) as a white solid. Mass spectrometry confirmed the product 732 (M+1). 1H NMR (300 MHz, Chloroform-*d*) δ 9.46 (s, 1H), 9.09 - 8.65 (m, 4H), 8.59 (m, 1H), 8.33 - 8.08 (m, 2H), 7.93 - 7.35 (m, 22H).

### Application Example 1

A glass substrate with 120 nm-thick indium-tin-oxide (ITO) transparent electrode (manufactured by Geomatec Co., Ltd.) used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 Å/sec at about 10⁻⁶ -10⁻⁸ mbar. As a hole injection layer, 5 nm-thick of compound HI was applied. Then 100 nm-thick of compound A and 60 nm-thick compound B were applied as hole transporting layer 1 and hole transporting layer 2, respectively. Subsequently, a mixture of 5% by weight of an emitter compound (Compound PD), 47.5% by weight of a host (compound **8**) and 47.5% by weight of compound D are applied to form a 40 nm-thick phosphorescent-emitting layer. On the emitting layer, 30 nm-thick layer of coevaporated compound C and Liq at ratio 1:1 by weight is applied as an electron transport layer. Finally, 1 nm-thick Liq is deposited as an electron injection layer and 80 nm-thick Al is then deposited as a cathode to complete the device. The device is sealed with a glass lid and a getter in an inert nitrogen atmosphere with less than 1 ppm of water and oxygen.

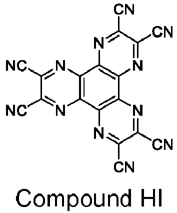

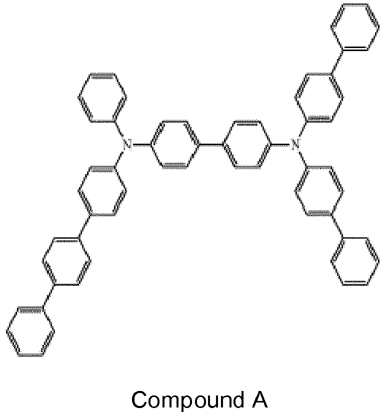

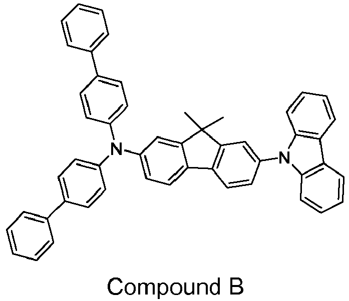

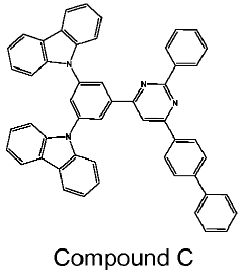

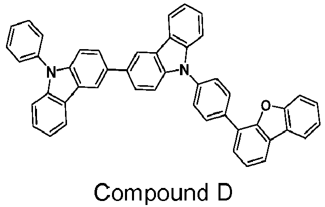

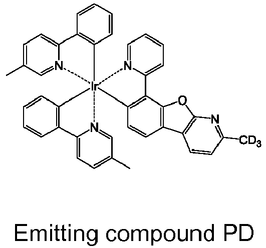

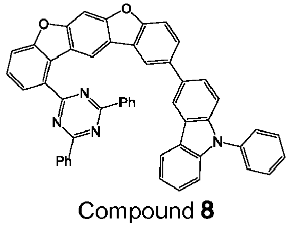

### OLED characterization

To characterize the OLED, electroluminescence spectra are recorded at various currents and voltages. In addition, the current-voltage characteristic is measured in combination with the luminance to determine luminous efficiency (L/J) and external quantum efficiency (EQE). Driving voltage U and current efficiency are given at a current density of 10mA/cm². Lifetime (LT80) of devices was measured at constant current of 50 mA/ cm². The device results are shown in Table 1.

**Table 1**

| **Appl. Ex.** | **Host** | **U [V]** | **L/J, cd/A** | **CIE(x,y)** | **LT80, h** |
|---|---|---|---|---|---|
| **Appl. Ex. 1** | **Compound 8** | **5.1** | **73.32** | **0.29/0.63** | **299** |

The results shown in Table 1 demonstrate that an inventive compound **8** can be used as a green host in an OLED.

## Claims

1. A polycyclic compound represented by one of the following formulae 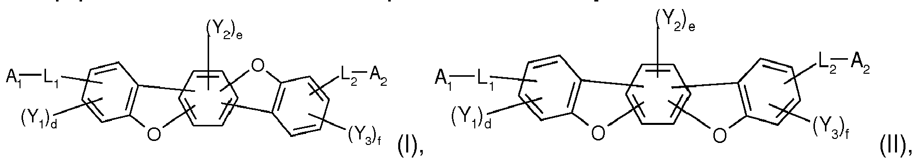 where:
L₁ represents a single bond, a substituted or unsubstituted alkylene group having 1 to 20 carbon atoms, a substituted or unsubstituted cycloalkylene group having a ring formed of 3 to 20 carbon atoms, a divalent silyl group or a divalent silyl group having 2 to 20 carbon atoms, a substituted or unsubstituted divalent aromatic hydrocarbon group having a ring formed of 6 to 24 carbon atoms, or a substituted or unsubstituted divalent aromatic heterocyclic group which has a ring formed of 3 to 24 atoms;
L₂ represents a single bond, a substituted or unsubstituted alkylene group having 1 to 20 carbon atoms, a substituted or unsubstituted cycloalkylene group having a ring formed of 3 to 20 carbon atoms, a divalent silyl group or a substituted divalent silyl group having 2 to 20 carbon atoms, a substituted or unsubstituted divalent aromatic hydrocarbon group having a ring formed of 6 to 24 carbon atoms, or a substituted or unsubstituted divalent aromatic heterocyclic group which has a ring formed of 3 to 24 atoms;
A₁ represents a group of formula (V) 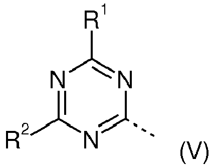 where:
R¹ and R² each independently represent hydrogen, a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms;
wherein the dotted line is a bonding site to L₁;
A₂ represents a group of formula (VI) or (VII) 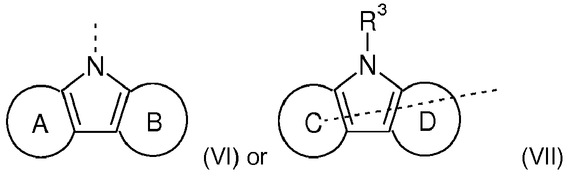 where
the ring A, the ring B, the ring C and the ring D each independently represent a substituted or unsubstituted monocyclic, bicyclic, or tricyclic carbocyclic ring system formed of 4 to 30 carbon atoms or a substituted or unsubstituted monocyclic, bicyclic, or tricyclic heterocyclic ring system formed of 3 to 30 atoms,
wherein the dotted line is a bonding site to L₂;
R³ represents a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms;
Y₁, Y₂, and Y₃ each independently represent CN, a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms;
d and f each represent a number of 0, 1, 2, or 3, and e represents a number of 0, 1, or 2.

2. The polycyclic compound according to claim 1, wherein
R¹ and R² in the group of formula (V) each independently represent a substituted or unsubstituted alkyl group having 1 to 10 carbon atoms, a substituted or unsubstituted cycloalkyl group having a ring formed of 3 to 10 carbon atoms, alkoxy group having 1 to 10 carbon atoms, a substituted or unsubstituted aralkyl group having 7 to 15 carbon atoms, a silyl group or a substituted silyl group having 3 to 10 carbon atoms, a substituted or unsubstituted aromatic hydrocarbon group having a ring formed of 6 to 18 carbon atoms, or a substituted or unsubstituted aromatic heterocyclic group which has a ring formed of 3 to 18 atoms; preferably, R¹ and R² each independently represent a substituted or unsubstituted aromatic hydrocarbon group having a ring formed of 6 to 18 carbon atoms, or a substituted or unsubstituted aromatic heterocyclic group which has a ring formed of 3 to 18 atoms, more preferably R¹ and R² each independently represent phenyl, biphenyl, terphenyl, naphthyl, phenanthrenyl, fluorenyl, dibenzofuranyl, dibenzothiophenyl, carbazolyl; most preferably R¹ and R² each independently represent phenyl, m-biphenyl, p-biphenyl, 1-naphthyl, 2-naphthyl 1-fluorenyl, 2-fluorenyl, 3-fluorenyl, 4-fluorenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl, 2-carbazolyl, 3-carbazolyl;
wherein the dotted line is a bonding site to L₁.

3. The polycyclic compound according to claim 1 or 2, wherein
the group of formula (V) is 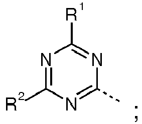 wherein
R¹ and R² each independently represent phenyl, m-biphenyl, p-biphenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl.

4. The polycyclic compound according to any one of claims 1 to 3, wherein
the ring A, the ring B, the ring C and the ring D in the group of formula (VI) or (VII) each independently represent 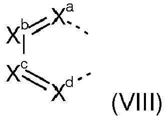 wherein
X^{a} is CR^{a} or N, preferably CR^{a},
X^{b} is CR^{b} or N, preferably CR^{b},
X^{c} is CR^{c} or N, preferably CR^{c},
X^{d} is CR^{d} or N, preferably CR^{d},
or 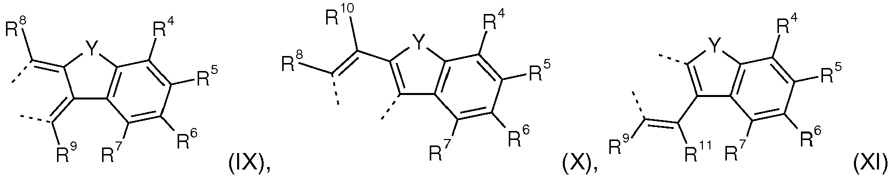 wherein
Y is NR¹², CR¹³R¹⁴, O or S,
R^{a}, R^{b}, R^{c}, R^{d}, R⁴, R⁵, R⁶, R⁷, R⁸, R⁹, R¹⁰ and R¹¹ each independently represent hydrogen, CN, a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, an aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms;
or
two adjacent groups R^{a}, R^{b}, R^{c}, R^{d}, R⁴, R⁵, R⁶, R⁷, R⁸, R⁹, R¹⁰ and R¹¹ may form together a carbocyclic or heterocyclic ring which may be unsubstituted or substituted;
R¹², R¹³ and R¹⁴ each independently represent a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms;
wherein in one of the rings C or D in formula (VII) one of R^{a}, R^{b}, R^{c} or R^{d}, R⁴, R⁵, R⁶ or R⁷ is a bonding site, wherein in the case that one of R^{a}, R^{b}, R^{c} or R^{d} is a bonding site, at least one of the groups X^{a}, X^{b}, X^{c} and X^{d} is CR^{a}, CR^{b}, CR^{c} or CR^{d}; and
wherein the dotted lines are bonding sites.

5. The polycyclic compound according to any one of claims 1 to 4, wherein
A₂ represents a group of formula 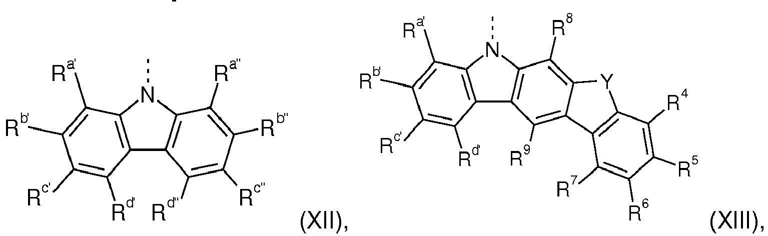 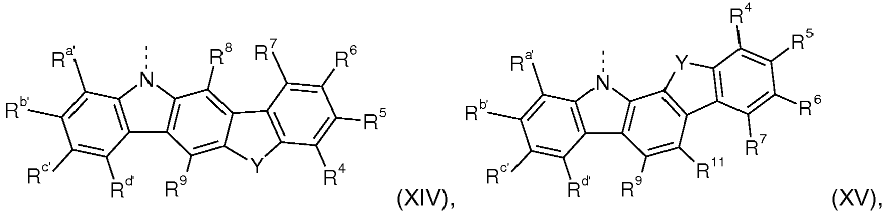 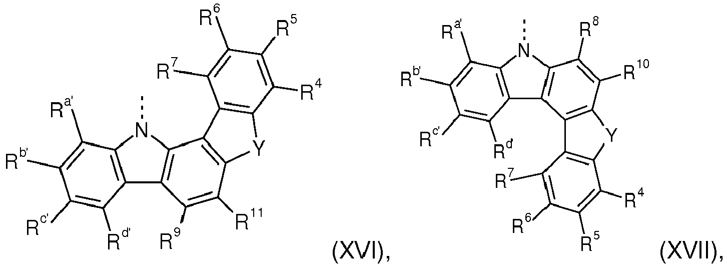 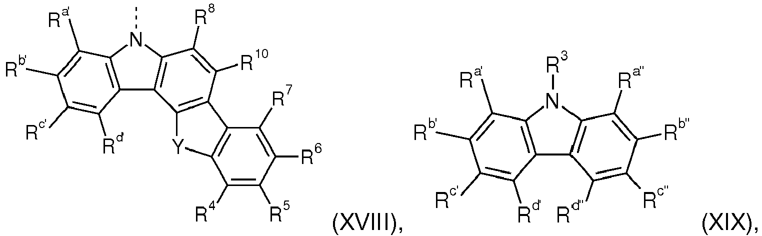 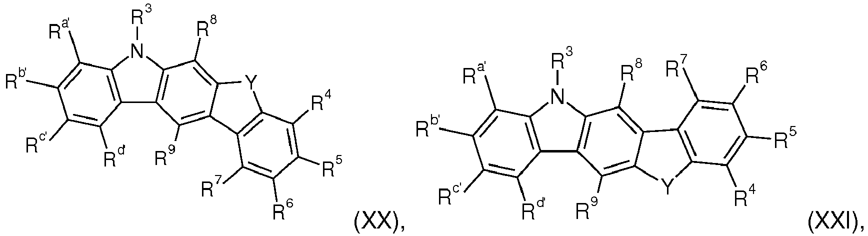 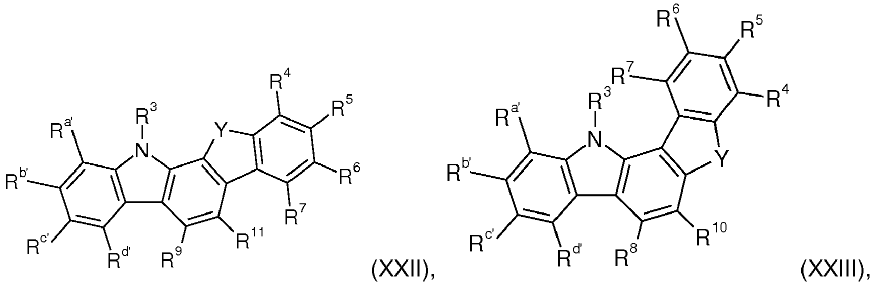 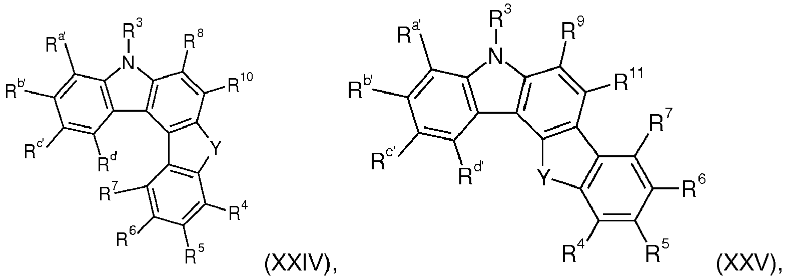 wherein
Y is NR¹², CR¹³R¹⁴, O or S,
R^{a'}, R^{b'}, R^{c'}, R^{d'}, R^{a"}, R^{b"}, R^{c"}, R^{d"}, R⁴, R⁵, R⁶, R⁷, R⁸, R⁹, R¹⁰ and R¹¹ each independently represent hydrogen, CN, a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms; or
two adjacent groups R^{a'}, R^{b'}, R^{c'}, R^{d'}, R^{a"}, R^{b"}, R^{c"}, R^{d"}, R⁴, R⁵, R⁶, R⁷, R⁸, R⁹, R¹⁰ and R¹¹ may form together a carbocyclic or heterocyclic ring which may be unsubstituted or substituted;
R¹², R¹³ and R¹⁴ each independently represent a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms;
wherein one of R^{a'}, R^{b'}, R^{c'}, R^{d'}, R^{a"}, R^{b"}, R^{c"} and R^{d"} in formula (XIX) is a bonding site,
wherein one of R^{a'}, R^{b'}, R^{c'}, R^{d'}, R⁴, R⁵, R⁶ and R⁷, preferably one of R^{a'}, R^{b'}, R^{c'}, R^{d'} in formulae (XX), (XXI), (XXII), (XXIII), (XXIV) and (XXV) is a bonding site; and
R³ represents a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms.

6. The polycyclic compound according to any one of claims 1 to 5, wherein the polycyclic compounds of formulae (I) and (II) each comprise a total number of substituents represented by Y¹, Y², and Y³ of 3 or less, i.e. the sum of d, e and f is 0, 1, 2 or 3.

7. The polycyclic compound according to any one of claims 1 to 6, wherein
L₁ represents a single bond or a phenylene group, preferably, L₁ is a single bond.

8. The polycyclic compound according to any one of claims 1 to 7, wherein
L₂ represents a single bond.

9. The polycyclic compound according to any one of claims 1 to 8, having one of the following formulae 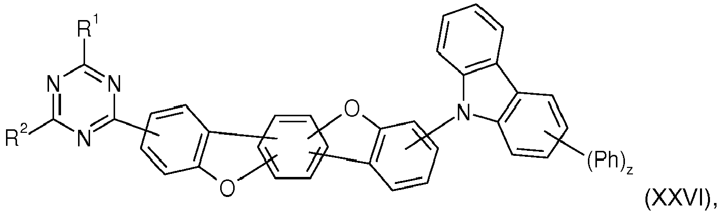 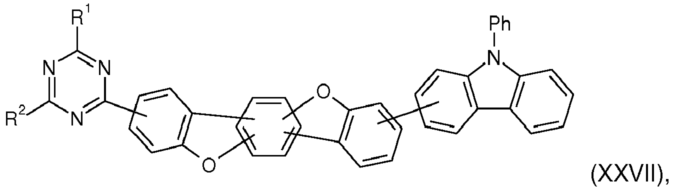 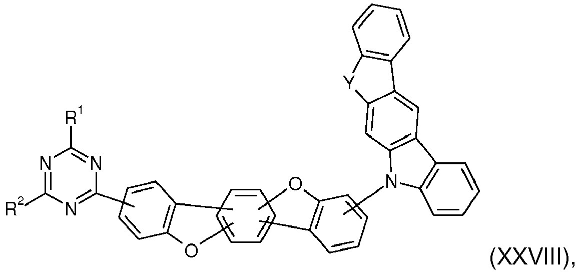 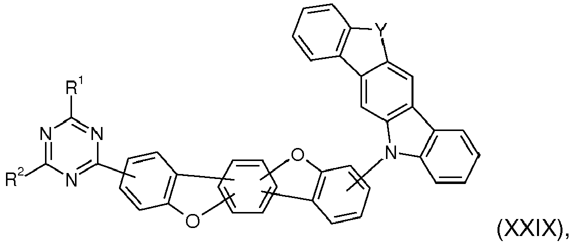 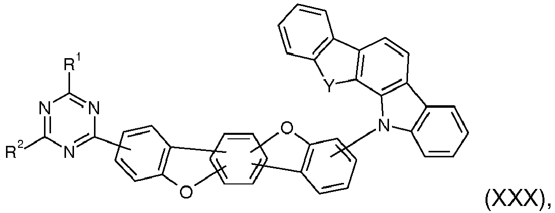 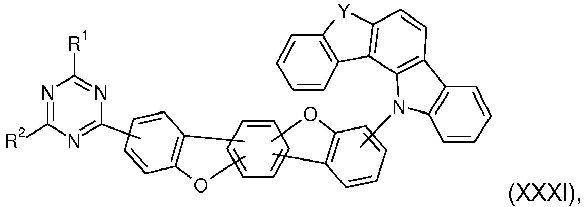 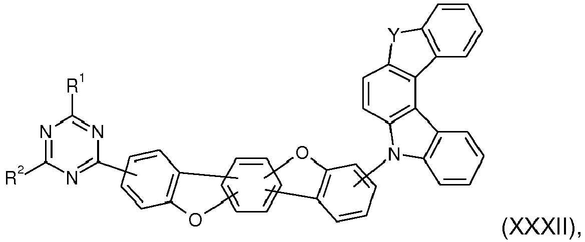 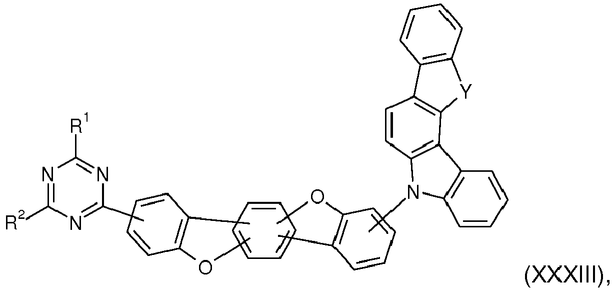 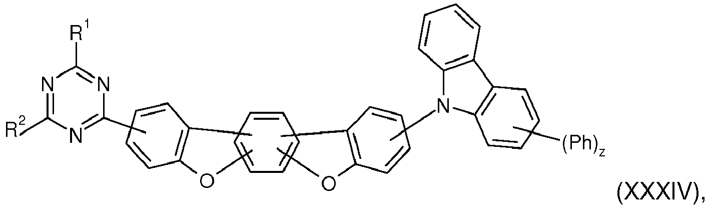 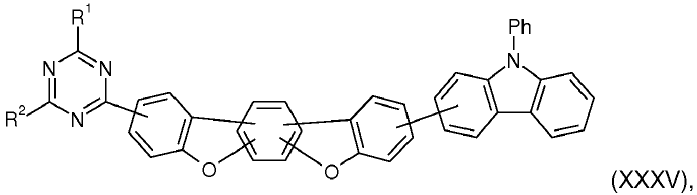 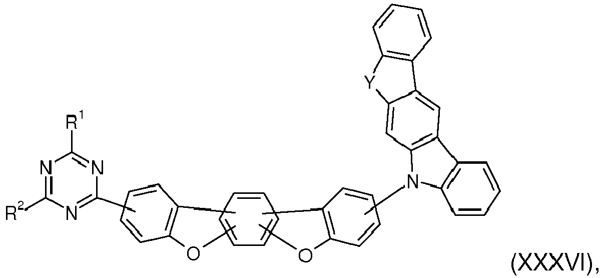 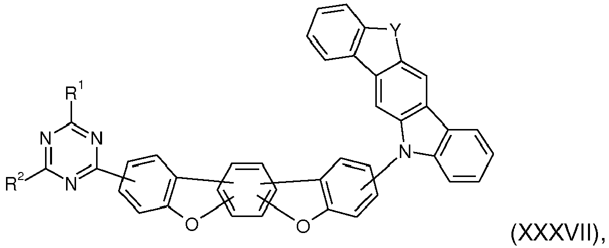 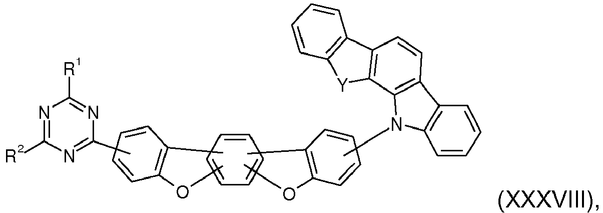 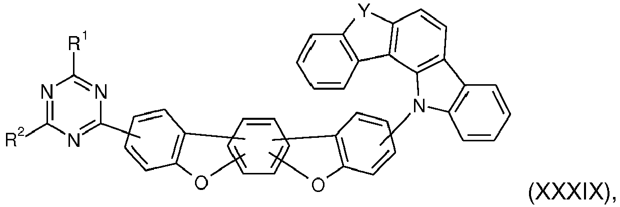 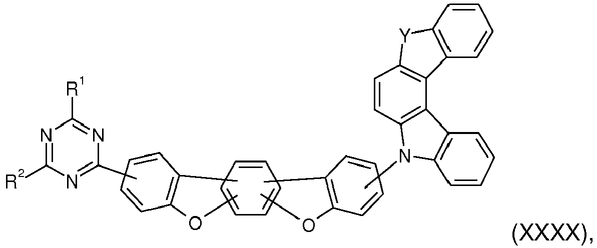 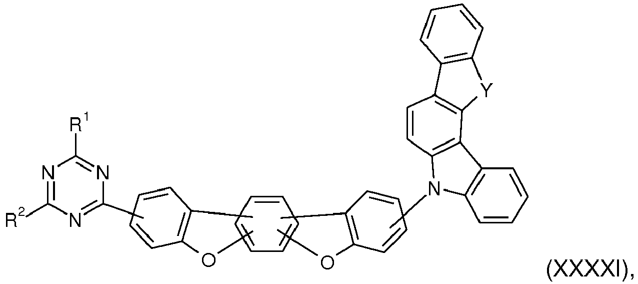 wherein
Y is NR¹², CR¹³R¹⁴, O or S, preferably N-Ph, C(CH₃)₂, S or O, and z is 0 or 1,
R¹², R¹³ and R¹⁴ each independently represent a substituted or unsubstituted alkyl group having 1 to 20 carbon atoms, a substituted or unsubstituted cycloalkyl group having a ring formed of 3 to 20 carbon atoms, a substituted or unsubstituted alkoxy group having 1 to 20 carbon atoms, a substituted or unsubstituted aralkyl group having 7 to 24 carbon atoms, a silyl group or a substituted silyl group having 3 to 20 carbon atoms, a substituted or unsubstituted aromatic hydrocarbon group having a ring formed of 6 to 24 carbon atoms, or a substituted or unsubstituted aromatic heterocyclic group which has a ring formed of 3 to 24 atoms;
R¹ and R² each independently represent phenyl, m-biphenyl, p-biphenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl;
Ph refers to a phenyl group,
preferably 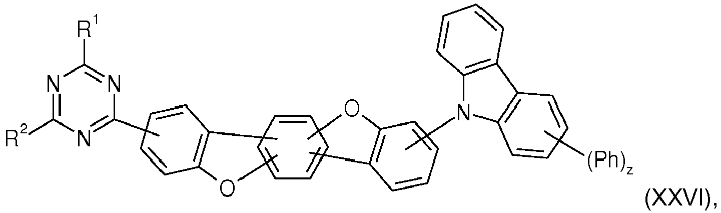 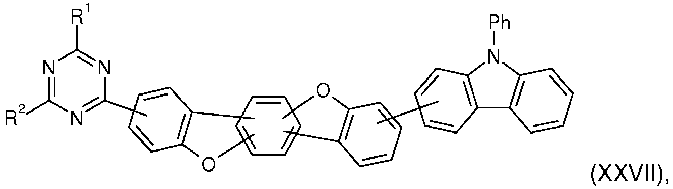 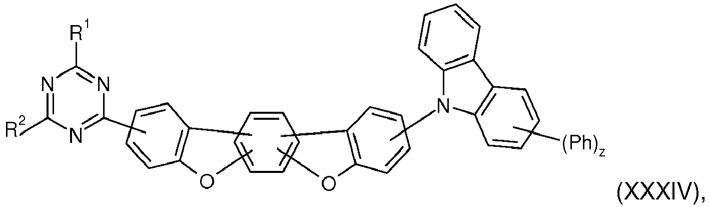 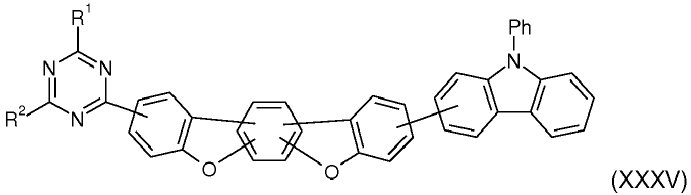 wherein
R¹ and R² each independently represent phenyl, m-biphenyl, p-biphenyl, 1-dibenzofuranyl, 2-dibenzofuranyl, 3-dibenzofuranyl, 4-dibenzofuranyl, 1-dibenzothiophenyl, 2-dibenzothiophenyl, 3-dibenzothiophenyl or 4-dibenzothiophenyl; and
z is 0 or 1.

10. An organic electroluminescence device comprising a cathode, an anode, and a plurality of organic layers provided between the cathode and the anode, wherein at least one of the plurality of organic layers is an emitting layer, wherein at least one organic layer comprises at least one compound as defined in any one of claims 1 to 9.

11. The organic electroluminescence device according to claim 10, wherein the emitting layer comprises the at least one compound as defined in any one of claims 1 to 9 as a host material.

12. The organic electroluminescence device according to claim 10 or 11, wherein the emitting layer comprises a heavy-metal complex material.

13. The organic electroluminescence device according to any one of claims 10 to 12, wherein the organic layers comprise an electron transporting layer comprising the at least one compound as defined in any one of claims 1 to 9, said electron transporting layer placed between the anode and the emitting layer.

14. The organic electroluminescence device according to claims 10 to 13, wherein at least one of the layers between the emitting layer and the anode comprises at least one selected from an alkali metal, an alkaline earth metal, a rare earth metal, a compound comprising an alkali metal, an alkaline earth metal, or a rare earth metal, and a complex comprising an alkali metal, an alkaline earth metal, or a rare earth metal.

15. Use of a compound as defined in any one of claims 1 to 9 in an organic electroluminescence device.
